# EUROPEAN PATENT APPLICATION

(11) **EP 3 957 315 A1**
(43) Date of publication of application: **23.02.2022**
(21) Application number: 21186345.1
(22) Date of filing: 14.09.2016
(51) Int. Cl.: A61K 35/50, C12N 5/0735, A61P 29/00

(54) **TREATMENT OF DIABETIC PERIPHERAL NEUROPATHY USING PLACENTAL CELLS**

(30) Priority: 15.09.2015 US 201562218885 P
(62) Divisional of application: 16847157.1
(71) Applicant: Celularity Inc., Florham Park, NJ 07932 (US)
(72) Inventor: FISCHKOFF, Steven A., New Jersey (US); CHITKARA, Denesh, New Jersey (US)
(74) Representative: Mewburn Ellis LLP

(57) **Abstract**

Provided herein are methods of using CD10⁺, CD34⁻, CD105⁺, CD200⁺ tissue culture plastic-adherent placental cells, e.g. placental stem cells, in the treatment of diabetic peripheral neuropathy (DPN).

## Description

This applications claims priority benefit of U.S. Provisional Patent Application No. 62/218,885, filed September 15, 2015, the contents of which are incorporated herein in their entirety.

### 1. FIELD

Provided herein are methods of using tissue culture plastic-adherent placental cells, e.g. placental stem cells, in the treatment of diabetic peripheral neuropathy (DPN).

### 2. BACKGROUND

The placenta is a particularly attractive source of stem cells. Because mammalian placentas are plentiful and are normally discarded as medical waste, they represent a unique source of medically-useful stem cells.

### 3. SUMMARY

Provided herein are methods of treating diabetic peripheral neuropathy (DPN) in a subject in need thereof, comprising administering to the subject a therapeutically effective amount of tissue culture plastic-adherent placental cells, e.g., placental stem cells, e.g., CD34⁻, CD10⁺, CD105⁺, CD200⁺ placental stem cells. In a specific embodiment, said placental cells are formulated as a pharmaceutical composition.

In a specific embodiment, a subject with DPN treated in accordance with the methods provided herein has type I diabetes. In another specific embodiment, a subject with DPN treated in accordance with the methods provided herein has type II diabetes. In certain embodiments, a subject treated in accordance with the methods provided herein has DPN in one or more of the arms, hands, legs, or feet. In certain embodiments, a subject treated in accordance with the methods provided herein has DPN in each of the arms, hands, legs, and feet.

In certain embodiments, a subject treated in accordance with the methods provided herein has DPN and also has a condition that causes a disruption in the flow of blood in the subject's peripheral vasculature. In a specific embodiment, the subject has peripheral arterial disease (PAD). In a specific embodiment, the subject has peripheral vascular disease.

In certain embodiments, the methods provided herein result in a detectable improvement of one or more symptoms of DPN in a subject treated in accordance with the methods provided herein. Exemplary symptoms of DPN include, without limitation, numbness or reduced ability to feel pain or temperature changes, a tingling or burning sensation in the limbs, sharp pains or cramps, increased sensitivity to touch, muscle weakness, loss of reflexes (e.g., in the ankle), loss of balance and/or coordination, foot problems (such as ulcers, infections, deformities, and bone and joint pain).

In certain embodiments, the methods provided herein comprise administering placental stem cells (e.g., a pharmaceutical composition comprising placental stem cells) to a subject having DPN in an amount and for a time sufficient for detectable improvement in one or more indicia of improvement. In a specific embodiment, said indicia of improvement is a change in the epidermal nerve fiber density following treatment, as compared to baseline. Epidermal nerve fiber density is a measurement used to assess the extent of peripheral diabetic neuropathy. To assess epidermal nerve fiber density, the number of nerve fibers in a skin biopsy is determined. An increase in the number/density of nerve fibers is indicative of improving neuropathy.

In certain embodiments, the methods provided herein comprise administering placental stem cells (e.g., a pharmaceutical composition comprising placental stem cells) to a subject having DPN in an amount and for a time sufficient for detectable improvement in quality of life of the subject as assessed by, e.g., (i) a 36-item Short Form Health Survey (SF-36) (see, e.g., Ware et al., Medical Care 30(6):473-483); (ii) the Diabetic peripheral neuropathy Scale Short Form (DFS-SF), which measures the impact of diabetic peripheral neuropathy on quality of life (see, e.g., Bann et al., Pharmacoeconomics, 2003, 21(17): 1277-90); (iii) the Patient Global Impression of Change Scale, to assess changes in neuropathy over time (see, e.g., Kamper et al., J. Man. Manip. Ther., 2009, 17(3): 163-170); and/or (iv) the EuroQol5D (EQ-5D^{™}) Scale, which is a health questionnaire used to obtain a descriptive profile and single index value for health status of a patient.

In a specific embodiment of the methods of treatment of DPN described herein, the placental cells (e.g., a pharmaceutical composition comprising placental stem cells) are administered by injection. In another specific embodiment of the methods of treatment of DPN described herein, the placental cells (e.g., a pharmaceutical composition comprising placental stem cells) are administered to a subject being treated by implantation in said subject of a matrix or scaffold comprising placental cells.

In a specific embodiment of the methods of treatment of DPN described herein, the placental cells (e.g., a pharmaceutical composition comprising placental stem cells) are administered intramuscularly. In another specific embodiment, the placental cells (e.g., a pharmaceutical composition comprising placental stem cells) are administered intramuscularly in the area of the DPN (e.g., in one or more of the legs, feet, arms, or hands). In another specific embodiment, the placental cells (e.g., a pharmaceutical composition comprising placental stem cells) are administered intramuscularly adjacent to the area of the DPN. In another specific embodiment, the placental cells (e.g., a pharmaceutical composition comprising placental stem cells) are administered below the knee and above the ankle of a subject that has been diagnosed with DPN. In another specific embodiment of the methods of treatment of DPN described herein, the placental cells (e.g., a pharmaceutical composition comprising placental stem cells) are administered intravenously. In another specific embodiment of the methods of treatment of DPN described herein, the placental cells (e.g., a pharmaceutical composition comprising placental stem cells) are administered subcutaneously. In another specific embodiment of the methods of treatment of DPN described herein, the placental cells (e.g., a pharmaceutical composition comprising placental stem cells) are administered locally. In another specific embodiment of the methods of treatment of DPN described herein, the placental cells (e.g., a pharmaceutical composition comprising placental stem cells) are administered systemically.

In certain embodiments, the methods of treatment of DPN described herein comprise administration of about 1 × 10³, 3 × 10³, 5 × 10³, 1 × 10⁴, 3 × 10⁴, 5 × 10⁴, 1 × 10⁵, 3 × 10⁵, 5 × 10⁵, 1 × 10⁶, 3 × 10⁶, 5 × 10⁶, 1 × 10⁷, 3 × 10⁷, 5 × 10⁷, 1 × 10⁸, 3 × 10⁸, 5 × 10⁸, 1 × 10⁹, 5 × 10⁹, or 1 × 10¹⁰ placental cells (e.g., as part of a pharmaceutical composition comprising placental stem cells). In certain embodiments, the methods of treatment of DPN described herein comprise administration of about 1 × 10³ to 3 × 10³, 3 × 10³ to 5 × 10³, 5 × 10³ to 1 × 10⁴, 1 × 10⁴ to 3 × 10⁴, 3 × 10⁴ to 5 × 10⁴, 5 × 10⁴ to 1 × 10⁵, 1 × 10⁵ to 3 × 10⁵, 3 × 10⁵, 1 to 5 × 10⁵, 5 × 10⁵, 1 to 1 × 10⁶, 1 × 10⁶ to 3 × 10⁶, 3 × 10⁶ to 5 × 10⁶, 5 × 10⁶ to 1 × 10⁷, 1 × 10⁷ to 3 × 10⁷, 3 × 10⁷ to 5 × 10⁷, 5 × 10⁷ to 1 × 10⁸, 1 × 10⁸ to 3 × 10⁸, 3 × 10⁸ to 5 × 10⁸, 5 × 10⁸ to 1 × 10⁹, 1 × 10⁹ to 5 × 10⁹, or 5 × 10⁹ to 1 × 10¹⁰ placental cells (e.g., as part of a pharmaceutical composition comprising placental stem cells). In a specific embodiment, the methods of treatment of DPN described herein comprise administration of about 3 × 10⁶ placental cells. In another specific embodiment, the methods of treatment of DPN described herein comprise administration of about 1 × 10⁷ placental cells. In another specific embodiment, the methods of treatment of DPN described herein comprise administration of about 3 × 10⁷ placental cells.

In a specific embodiment of the methods of treatment of DPN described herein, the placental stem cells (e.g., a pharmaceutical composition comprising placental stem cells) are administered intramuscularly to a subject more than once, with one week between administrations, e.g., placental cells are administered on day 1 (the first day of administration) and a second dose of placental stem cells (e.g., a pharmaceutical composition comprising placental stem cells) is administered one week later (i.e., on day 8). In another specific embodiment, the methods comprise administration of about 3 × 10⁶ placental stem cells on each day of administration (i.e., on days 1 and 8). In another specific embodiment, the methods comprise administration of about 3 × 10⁷ placental cells on each day of administration (i.e., on days 1 and 8). In another specific embodiment, the placental cells are administered to a subject on at least three different occasions, with about one week between administrations.

In another specific embodiment of the methods of treatment of DPN described herein, the placental stem cells (e.g., a pharmaceutical composition comprising placental stem cells) are administered to a subject more than once, with one month between administrations, e.g., placental cells are administered on day 1 (the first day of administration) and a second dose of placental stem cells (e.g., a pharmaceutical composition comprising placental stem cells) is administered about one month later (e.g., on day 27, 28, 29, 30, 31, 32, or 33). In a specific embodiment, the methods comprise administration of about 3 × 10⁶ placental stem cells on each day of administration (e.g., 3 × 10⁶ placental stem cells are administered on day 1, and about 3 × 10⁶ placental stem cells are administered about 1 month after day 1, e.g., on day 27, 28, 29, 30, 31, 32, or 33). In another specific embodiment, the methods comprise administration of about 3 × 10⁷ placental cells on each day of administration (e.g., 3 × 10⁷ placental stem cells are administered on day 1, and about 3 × 10⁷ placental stem cells are administered about 1 month after day 1, e.g., on day 27, 28, 29, 30, 31, 32, or 33). In another specific embodiment, the placental cells are administered are administered to a subject on at least three different occasions, with about one month between administrations.

In certain embodiments, the dose of placental cells (e.g., a pharmaceutical composition comprising placental stem cells) is administered using multiple different injections, e.g., a single dose of placental cells (e.g., a dose comprising about 3 × 10⁶ placental stem cells or a dose comprising about 3 × 10⁷ placental stem cells) is administered by injecting the subject being treated multiple times. In certain embodiments, a dose of placental cells (e.g., a dose comprising about 3 × 10⁶ placental stem cells or a dose comprising about 3 × 10⁷ placental stem cells) is administered across 2, 3, 4, 5, 6, 7, 8, 9, 10, 11, 12, 13, 14, 15, 16, 17, 18, 19, or 20 injections. The individual injections that make up administration of single dose of placental cells can comprise, in certain embodiments, 0.1 ml of placental cells (or a pharmaceutical composition thereof), 0.2 ml of placental cells (or a pharmaceutical composition thereof), 0.3 ml of placental cells (or a pharmaceutical composition thereof), 0.4 ml of placental cells (or a pharmaceutical composition thereof), or 0.5 ml of placental cells (or a pharmaceutical composition thereof). In a specific embodiment, the individual injections that in total make up a single dose of placental cells (e.g., a dose comprising about 3 × 10⁶ placental stem cells or a dose comprising about 3 × 10⁷ placental stem cells) comprise 0.3 ml of placental cells.

In a specific embodiment, a dose of placental cells (e.g., a dose comprising about 3 × 10⁶ placental stem cells or a dose comprising about 3 × 10⁷ placental stem cells) is administered across 15 separate injections, wherein each injection contains 0.3 ml of a solution comprising placental cells (or a pharmaceutical composition thereof). In a specific embodiment, said administration is intramuscular. In another specific embodiment, said administration is intramuscular, and the intramuscular injections are administered below the knee and above the ankle of the subject having DPN.

In a specific embodiment, provided herein is a method of treating DPN in a subject, said method comprising administering to said subject a dose of about 3 × 10⁶ placental stem cells, wherein (i) said placental stem cells are administered intramuscularly on about a monthly basis for at least three months (e.g., placental stem cells are administered on day 1; on day 27, 28, 29, 30, 31, or 32; and on day 56, 57, 58, 59, 60, 61, 62, or 63), (ii) each dose is administered across 15 separate injections, wherein each injection contains 0.3 ml of a solution comprising placental cells (or a pharmaceutical composition thereof), and (iii) said intramuscular injections are administered below the knee and above the ankle of the subject having DPN.

In a specific embodiment, provided herein is a method of treating DPN in a subject, said method comprising administering to said subject a dose of about 3 × 10⁷ placental stem cells, wherein (i) said placental stem cells are administered intramuscularly on about a monthly basis for at least three months (e.g., placental stem cells are administered on day 1; on day 27, 28, 29, 30, 31, or 32; and on day 56, 57, 58, 59, 60, 61, 62, or 63), (ii) each dose is administered across 15 separate injections, wherein each injection contains 0.3 ml of a solution comprising placental cells (or a pharmaceutical composition thereof), and (iii) said intramuscular injections are administered below the knee and above the ankle of the subject having DPN.

The placental cells used in the methods described herein adhere to tissue culture plastic and are CD34⁻, CD10⁺, CD105⁺ and CD200⁺, as detectable by, e.g., flow cytometry. Further characteristics of the placental cells used in the methods provided herein are described in Section 4.1. Compositions, e.g., pharmaceutical compositions, comprising the placental stem cells to be used in the methods provided herein are described in Section 4.3.

### 3.1 Definitions

As used herein, the term "about," when referring to a stated numeric value, indicates a value within plus or minus 10% of the stated numeric value.

As used herein, the term "derived" means isolated from or otherwise purified. For example, placental derived adherent cells are isolated from placenta. The term "derived" encompasses cells that are cultured from cells isolated directly from a tissue, *e.g.,* the placenta, and cells cultured or expanded from primary isolates.

As used herein, the term "isolated cell," *e.g.,* "isolated placental cell," "isolated placental stem cell," and the like, means a cell that is substantially separated from other, different cells of the tissue, e.g., placenta, from which the stem cell is derived. A cell is "isolated" if at least 50%, 60%, 70%, 80%, 90%, 95%, or at least 99% of the cells, e.g., non-stem cells, with which the stem cell is naturally associated, or stem cells displaying a different marker profile, are removed from the stem cell, *e.g.,* during collection and/or culture of the stem cell.

As used herein, the term "population of isolated cells" means a population of cells that is substantially separated from other cells of a tissue, e.g., placenta, from which the population of cells is derived.

As used herein, the term "placental cell" refers to a stem cell or progenitor cell that is isolated from a mammalian placenta, *e.g.,* as described in Section 4.1, below, or cultured from cells isolated from a mammalian placenta, either as a primary isolate or a cultured cell, regardless of the number of passages after a primary culture. In certain embodiments, the term "placental cells," as used herein does not, however, refer to trophoblasts, cytotrophoblasts, syncitiotrophoblasts, angioblasts, hemangioblasts, embryonic germ cells, embryonic stem cells, cells obtained from an inner cell mass of a blastocyst, or cells obtained from a gonadal ridge of a late embryo, e.g., an embryonic germ cell.

As used herein, a placental cell is "positive" for a particular marker when that marker is detectable above background. Detection of a particular marker can, for example, be accomplished either by use of antibodies, or by oligonucleotide probes or primers based on the sequence of the gene or mRNA encoding the marker. For example, a placental cell is positive for, *e.g.,* CD73 because CD73 is detectable on placental cells in an amount detectably greater than background (in comparison to, *e.g.,* an isotype control). A cell is also positive for a marker when that marker can be used to distinguish the cell from at least one other cell type, or can be used to select or isolate the cell when present or expressed by the cell. In the context of, *e.g.,* antibody-mediated detection, "positive," as an indication a particular cell surface marker is present, means that the marker is detectable using an antibody, e.g., a fluorescently-labeled antibody, specific for that marker; "positive" also refers to a cell exhibiting the marker in an amount that produces a signal, *e.g.,* in a cytometer, that is detectably above background. For example, a cell is "CD200⁺" where the cell is detectably labeled with an antibody specific to CD200, and the signal from the antibody is detectably higher than that of a control (*e.g.,* background or an isotype control). Conversely, "negative" in the same context means that the cell surface marker is not detectable using an antibody specific for that marker compared a control (e.g., background or an isotype control). For example, a cell is "CD34⁻" where the cell is not reproducibly detectably labeled with an antibody specific to CD34 to a greater degree than a control (e.g., background or an isotype control). Markers not detected, or not detectable, using antibodies are determined to be positive or negative in a similar manner, using an appropriate control. For example, a cell or population of cells can be determined to be OCT-4⁺ if the amount of OCT-4 RNA detected in RNA from the cell or population of cells is detectably greater than background as determined, *e.g.,* by a method of detecting RNA such as RT-PCR, slot blots, *etc.* Unless otherwise noted herein, cluster of differentiation ("CD") markers are detected using antibodies. In certain embodiments, OCT-4 is determined to be present, and a cell is "OCT-4⁺" if OCT-4 is detectable using RT-PCR.

As used herein, the terms "subject," "patient," and "individual" may be used interchangeably to refer to a mammal being treated with a method of treatment described herein. In a specific embodiment the subject to be treated is a human.

### 4. DETAILED DESCRIPTION

Provided herein are methods of treating diabetic peripheral neuropathy (DPN) in a subject in need thereof, comprising administering to the subject a therapeutically effective amount of tissue culture plastic-adherent placental cells, e.g., placental stem cells, e.g., CD34⁻, CD10⁺, CD105⁺, CD200⁺ placental stem cells. In a specific embodiment, said placental cells are formulated as a pharmaceutical composition.

In a specific embodiment, a subject with DPN treated in accordance with the methods provided herein has type I diabetes. In another specific embodiment, a subject with DPN treated in accordance with the methods provided herein has type II diabetes. In certain embodiments, a subject treated in accordance with the methods provided herein has DPN in one or more of the arms, hands, legs, or feet. In certain embodiments, a subject treated in accordance with the methods provided herein has DPN in each of the arms, hands, legs, and feet.

In certain embodiments, a subject treated in accordance with the methods provided herein has DPN and also has a condition that causes a disruption in the flow of blood in the subject's peripheral vasculature. In a specific embodiment, the subject has peripheral arterial disease (PAD). In a specific embodiment, the subject has peripheral vascular disease.

In certain embodiments, the methods provided herein result in a detectable improvement of one or more symptoms of DPN in a subject treated in accordance with the methods provided herein. Exemplary symptoms of DPN include, without limitation, numbness or reduced ability to feel pain or temperature changes, a tingling or burning sensation in the limbs, sharp pains or cramps, increased sensitivity to touch, muscle weakness, loss of reflexes (e.g., in the ankle), loss of balance and/or coordination, foot problems (such as ulcers, infections, deformities, and bone and joint pain).

In certain embodiments, the methods provided herein comprise administering placental stem cells (e.g., a pharmaceutical composition comprising placental stem cells) to a subject having DPN in an amount and for a time sufficient for detectable improvement in one or more indicia of improvement. In a specific embodiment, said indicia of improvement is a change in the epidermal nerve fiber density following treatment, as compared to baseline. Epidermal nerve fiber density is a measurement used to assess the extent of peripheral diabetic neuropathy. To assess epidermal nerve fiber density, the number of nerve fibers in a skin biopsy is determined. An increase in the number/density of nerve fibers is indicative of improving neuropathy.

In certain embodiments, the methods provided herein comprise administering placental stem cells (e.g., a pharmaceutical composition comprising placental stem cells) to a subject having DPN in an amount and for a time sufficient for detectable improvement in quality of life of the subject as assessed by, e.g., (i) a 36-item Short Form Health Survey (SF-36) (see, e.g., Ware et al., Medical Care 30(6):473-483); (ii) the Diabetic peripheral neuropathy Scale Short Form (DFS-SF), which measures the impact of diabetic peripheral neuropathy on quality of life (see, e.g., Bann et al., Pharmacoeconomics, 2003, 21(17): 1277-90); (iii) the Patient Global Impression of Change Scale, to assess changes in neuropathy over time (see, e.g., Kamper et al., J. Man. Manip. Ther., 2009, 17(3): 163-170); and/or (iv) the EuroQol5D (EQ-5D^{™}) Scale, which is a health questionnaire used to obtain a descriptive profile and single index value for health status of a patient..

In a specific embodiment of the methods of treatment of DPN described herein, the placental cells (e.g., a pharmaceutical composition comprising placental stem cells) are administered by injection. In another specific embodiment of the methods of treatment of DPN described herein, the placental cells (e.g., a pharmaceutical composition comprising placental stem cells) are administered to a subject being treated by implantation in said subject of a matrix or scaffold comprising placental cells.

In a specific embodiment of the methods of treatment of DPN described herein, the placental cells (e.g., a pharmaceutical composition comprising placental stem cells) are administered intramuscularly. In another specific embodiment, the placental cells (e.g., a pharmaceutical composition comprising placental stem cells) are administered intramuscularly in the area of the DPN (e.g., in one or more of the legs, feet, arms, or hands). In another specific embodiment, the placental cells (e.g., a pharmaceutical composition comprising placental stem cells) are administered intramuscularly adjacent to the area of the DPN. In another specific embodiment, the placental cells (e.g., a pharmaceutical composition comprising placental stem cells) are administered below the knee and above the ankle of a subject that has been diagnosed with DPN. In another specific embodiment of the methods of treatment of DPN described herein, the placental cells (e.g., a pharmaceutical composition comprising placental stem cells) are administered intravenously. In another specific embodiment of the methods of treatment of DPN described herein, the placental cells (e.g., a pharmaceutical composition comprising placental stem cells) are administered subcutaneously. In another specific embodiment of the methods of treatment of DPN described herein, the placental cells (e.g., a pharmaceutical composition comprising placental stem cells) are administered locally. In another specific embodiment of the methods of treatment of DPN described herein, the placental cells (e.g., a pharmaceutical composition comprising placental stem cells) are administered systemically.

In certain embodiments, the methods of treatment of DPN described herein comprise administration of about 1 × 10³, 3 × 10³, 5 × 10³, 1 × 10⁴, 3 × 10⁴, 5 × 10⁴, 1 × 10⁵, 3 × 10⁵, 5 × 10⁵, 1 × 10⁶, 3 × 10⁶, 5 × 10⁶, 1 × 10⁷, 3 × 10⁷, 5 × 10⁷, 1 × 10⁸, 3 × 10⁸, 5 × 10⁸ 1 × 10⁹, 5 × 10⁹, or 1 × 10¹⁰ placental cells (e.g., as part of a pharmaceutical composition comprising placental stem cells). In certain embodiments, the methods of treatment of DPN described herein comprise administration of about 1 × 10³ to 3 × 10³, 3 × 10³ to 5 × 10³, 5 × 10³ to 1 × 10⁴, 1 × 10⁴ to 3 × 10⁴, 3 × 10⁴ to 5 × 10⁴, 5 × 10⁴ to 1 × 10⁵, 1 × 10⁵ to 3 × 10⁵, 3 × 10⁵, 1 to 5 × 10⁵, 5 × 10⁵, 1 to 1 × 10⁶, 1 × 10⁶ to 3 × 10⁶, 3 × 10⁶ to 5 × 10⁶, 5 × 10⁶ to 1 × 10⁷, 1 × 10⁷ to 3 × 10⁷, 3 × 10⁷ to 5 × 10⁷, 5 × 10⁷ to 1 × 10⁸, 1 × 10⁸ to 3 × 10⁸, 3 × 10⁸ to 5 × 10⁸, 5 × 10⁸ to 1 × 10⁹, 1 × 10⁹ to 5 × 10⁹, or 5 × 10⁹ to 1 × 10¹⁰ placental cells (e.g., as part of a pharmaceutical composition comprising placental stem cells). In a specific embodiment, the methods of treatment of DPN described herein comprise administration of about 3 × 10⁶ placental cells. In another specific embodiment, the methods of treatment of DPN described herein comprise administration of about 1 × 10⁷ placental cells. In another specific embodiment, the methods of treatment of DPN described herein comprise administration of about 3 × 10⁷ placental cells.

In a specific embodiment of the methods of treatment of DPN described herein, the placental stem cells (e.g., a pharmaceutical composition comprising placental stem cells) are administered intramuscularly to a subject more than once, with one week between administrations, e.g., placental cells are administered on day 1 (the first day of administration) and a second dose of placental stem cells (e.g., a pharmaceutical composition comprising placental stem cells) is administered one week later (i.e., on day 8). In another specific embodiment, the methods comprise administration of about 3 × 10⁶ placental stem cells on each day of administration (i.e., on days 1 and 8). In another specific embodiment, the methods comprise administration of about 3 × 10⁷ placental cells on each day of administration (i.e., on days 1 and 8). In another specific embodiment, the placental cells are administered to a subject on at least three different occasions, with about one week between administrations.

In another specific embodiment of the methods of treatment of DPN described herein, the placental stem cells (e.g., a pharmaceutical composition comprising placental stem cells) are administered to a subject more than once, with one month between administrations, e.g., placental cells are administered on day 1 (the first day of administration) and a second dose of placental stem cells (e.g., a pharmaceutical composition comprising placental stem cells) is administered about one month later (e.g., on day 27, 28, 29, 30, 31, 32, or 33). In a specific embodiment, the methods comprise administration of about 3 × 10⁶ placental stem cells on each day of administration (e.g., 3 × 10⁶ placental stem cells are administered on day 1, and about 3 × 10⁶ placental stem cells are administered about 1 month after day 1, e.g., on day 27, 28, 29, 30, 31, 32, or 33). In another specific embodiment, the methods comprise administration of about 3 × 10⁷ placental cells on each day of administration (e.g., 3 × 10⁷ placental stem cells are administered on day 1, and about 3 × 10⁷ placental stem cells are administered about 1 month after day 1, e.g., on day 27, 28, 29, 30, 31, 32, or 33). In another specific embodiment, the placental cells are administered are administered to a subject on at least three different occasions, with about one month between administrations.

In certain embodiments, the dose of placental cells (e.g., a pharmaceutical composition comprising placental stem cells) is administered using multiple different injections, e.g., a single dose of placental cells (e.g., a dose comprising about 3 × 10⁶ placental stem cells or a dose comprising about 3 × 10⁷ placental stem cells) is administered by injecting the subject being treated multiple times. In certain embodiments, a dose of placental cells (e.g., a dose comprising about 3 × 10⁶ placental stem cells or a dose comprising about 3 × 10⁷ placental stem cells) is administered across 2, 3, 4, 5, 6, 7, 8, 9, 10, 11, 12, 13, 14, 15, 16, 17, 18, 19, or 20 injections. The individual injections that make up administration of single dose of placental cells can comprise, in certain embodiments, 0.1 ml of placental cells (or a pharmaceutical composition thereof), 0.2 ml of placental cells (or a pharmaceutical composition thereof), 0.3 ml of placental cells (or a pharmaceutical composition thereof), 0.4 ml of placental cells (or a pharmaceutical composition thereof), or 0.5 ml of placental cells (or a pharmaceutical composition thereof). In a specific embodiment, the individual injections that in total make up a single dose of placental cells (e.g., a dose comprising about 3 × 10⁶ placental stem cells or a dose comprising about 3 × 10⁷ placental stem cells) comprise 0.3 ml of placental cells.

In a specific embodiment, a dose of placental cells (e.g., a dose comprising about 3 × 10⁶ placental stem cells or a dose comprising about 3 × 10⁷ placental stem cells) is administered across 15 separate injections, wherein each injection contains 0.3 ml of a solution comprising placental cells (or a pharmaceutical composition thereof). In a specific embodiment, said administration is intramuscular. In another specific embodiment, said administration is intramuscular, and the intramuscular injections are administered below the knee and above the ankle of the subject having DPN.

In a specific embodiment, provided herein is a method of treating DPN in a subject, said method comprising administering to said subject a dose of about 3 × 10⁶ placental stem cells, wherein (i) said placental stem cells are administered intramuscularly on about a monthly basis for at least three months (e.g., placental stem cells are administered on day 1; on day 27, 28, 29, 30, 31, or 32; and on day 56, 57, 58, 59, 60, 61, 62, or 63), (ii) each dose is administered across 15 separate injections, wherein each injection contains 0.3 ml of a solution comprising placental cells (or a pharmaceutical composition thereof), and (iii) said intramuscular injections are administered below the knee and above the ankle of the subject having DPN.

In a specific embodiment, provided herein is a method of treating DPN in a subject, said method comprising administering to said subject a dose of about 3 × 10⁷ placental stem cells, wherein (i) said placental stem cells are administered intramuscularly on about a monthly basis for at least three months (e.g., placental stem cells are administered on day 1; on day 27, 28, 29, 30, 31, or 32; and on day 56, 57, 58, 59, 60, 61, 62, or 63), (ii) each dose is administered across 15 separate injections, wherein each injection contains 0.3 ml of a solution comprising placental cells (or a pharmaceutical composition thereof), and (iii) said intramuscular injections are administered below the knee and above the ankle of the subject having DPN.

The placental cells used in the methods described herein adhere to tissue culture plastic and are CD34⁻, CD10⁺, CD105⁺ and CD200⁺, as detectable by, e.g., flow cytometry. Further characteristics of the placental cells used in the methods provided herein are described in Section 4.1. Compositions, e.g., pharmaceutical compositions, comprising the placental stem cells to be used in the methods provided herein are described in Section 4.3.

### 4.1 ISOLATED PLACENTAL CELLS AND ISOLATED PLACENTAL CELL POPULATIONS

The isolated placental cells, sometimes referred to herein as PDACs (and also sometimes designated "PDA-002"), useful in the methods of treatment of DPN provided herein are obtainable from a placenta or part thereof, adhere to a tissue culture substrate and have characteristics of multipotent cells or stem cells, but are not trophoblasts. In certain embodiments, the isolated placental cells useful in the methods disclosed herein have the capacity to differentiate into non-placental cell types.

The isolated placental cells useful in the methods disclosed herein can be either fetal or maternal in origin (that is, can have the genotype of either the fetus or mother, respectively). Preferably, the isolated placental cells and populations of isolated placental cells are fetal in origin. As used herein, the phrase "fetal in origin" or "non-maternal in origin" indicates that the isolated placental cells or populations of isolated placental cells are obtained from the umbilical cord or placental structures associated with the fetus, *i.e.,* that have the fetal genotype. As used herein, the phrase "maternal in origin" indicates that the cells or populations of cells are obtained from a placental structures associated with the mother, e.g., which have the maternal genotype. Isolated placental cells, *e.g.,* PDACs, or populations of cells comprising the isolated placental cells, can comprise isolated placental cells that are solely fetal or maternal in origin, or can comprise a mixed population of isolated placental cells of both fetal and maternal origin. The isolated placental cells, and populations of cells comprising the isolated placental cells, can be identified and selected by the morphological, marker, and culture characteristics discussed below. In certain embodiments, any of the placental cells, *e.g.,* placental stem cells or placental multipotent cells described herein, are autologous to a recipient, e.g., an individual who has a DPN. In certain other embodiments, any of the placental cells, *e.g.,* placental stem cells or placental multipotent cells described herein, are heterologous to a recipient, e.g., an individual who has a DPN.

### 4.1.1 Physical and Morphological Characteristics

The isolated placental cells described herein (PDACs), when cultured in primary cultures or in cell culture, adhere to the tissue culture substrate, *e.g.,* tissue culture container surface (*e.g.,* tissue culture plastic), or to a tissue culture surface coated with extracellular matrix or ligands such as laminin, collagen (e.g., native or denatured), gelatin, fibronectin, ornithine, vitronectin, and extracellular membrane protein (e.g., MATRIGEL^{®} (BD Discovery Labware, Bedford, Mass.)). The isolated placental cells in culture assume a generally fibroblastoid, stellate appearance, with a number of cytoplasmic processes extending from the central cell body. The cells are, however, morphologically distinguishable from fibroblasts cultured under the same conditions, as the isolated placental cells exhibit a greater number of such processes than do fibroblasts. Morphologically, isolated placental cells are also distinguishable from hematopoietic stem cells, which generally assume a more rounded, or cobblestone, morphology in culture.

In certain embodiments, the isolated placental cells useful in the methods disclosed herein, when cultured in a growth medium, develop embryoid-like bodies. Embryoid-like bodies are noncontiguous clumps of cells that can grow on top of an adherent layer of proliferating isolated placental cells. The term "embryoid-like" is used because the clumps of cells resemble embryoid bodies, clumps of cells that grow from cultures of embryonic stem cells. Growth medium in which embryoid-like bodies can develop in a proliferating culture of isolated placental cells includes medium comprising, *e.g.,* DMEM-LG *(e.g.,* from Gibco); 2% fetal calf serum (e.g., from Hyclone Labs.); 1x insulin-transferrin-selenium (ITS); 1x linoleic acid-bovine serum albumin (LA-BSA); 10⁻⁹ M dexamethasone (e.g., from Sigma); 10⁻⁴ M ascorbic acid 2-phosphate *(e.g.,* from Sigma); epidermal growth factor 10 ng/mL *(e.g.,* from R&D Systems); and platelet-derived growth factor (PDGF-BB) 10 ng/mL *(e.g.,* from R&D Systems).

### 4.1.2 Cell Surface, Molecular and Genetic Markers

The isolated placental cells, *e.g.,* isolated multipotent placental cells or isolated placental stem cells, and populations of such isolated placental cells, useful in the methods disclosed herein, *e.g.,* the methods of treatment of a DPN of a subject, are tissue culture plastic-adherent human placental cells that have characteristics of multipotent cells or stem cells, and express a plurality of markers that can be used to identify and/or isolate the cells, or populations of cells that comprise the stem cells. In certain embodiments, the PDACs are angiogenic, *e.g., in vitro* or *in vivo.* The isolated placental cells, and placental cell populations described herein (that is, two or more isolated placental cells) include placental cells and placental cell-containing cell populations obtained directly from the placenta, or any part thereof (e.g., chorion, placental cotyledons, or the like). Isolated placental cell populations also include populations of (that is, two or more) isolated placental cells in culture, and a population in a container, e.g., a bag. The isolated placental cells described herein are not bone marrow-derived mesenchymal cells, adipose-derived mesenchymal stem cells, or mesenchymal cells obtained from umbilical cord blood, placental blood, or peripheral blood. Placental cells, *e.g.,* placental multipotent cells and placental cells, useful in the methods and compositions described herein are described herein and, *e.g.,* in U.S. Patent Nos. 7,311,904; 7,311,905; and 7,468,276; and in U.S. Patent Application Publication No. 2007/0275362, the disclosures of which are hereby incorporated by reference in their entireties.

In certain embodiments, the isolated placental cells are isolated placental stem cells. In certain other embodiments, the isolated placental cells are isolated placental multipotent cells. In one embodiment, the isolated placental cells, *e.g,* PDACs, are CD34⁻, CD10⁺ and CD105⁺ as detected by flow cytometry. In another specific embodiment, the isolated CD34⁻, CD10⁺, CD105⁺ placental cells have the potential to differentiate into cells of a neural phenotype, cells of an osteogenic phenotype, and/or cells of a chondrogenic phenotype. In another specific embodiment, the isolated CD34⁻, CD10⁺, CD105⁺ placental cells are additionally CD200⁺. In another specific embodiment, the isolated CD34⁻, CD10⁺, CD105⁺ placental cells are additionally CD45⁻ or CD90⁺. In another specific embodiment, the isolated CD34⁻, CD10⁺, CD105⁺ placental cells are additionally CD45⁻ and CD90⁺, as detected by flow cytometry. In another specific embodiment, the isolated CD34⁻, CD10⁺, CD105⁺, CD200⁺ placental cells are additionally CD90⁺ or CD45⁻, as detected by flow cytometry. In another specific embodiment, the isolated CD34⁻, CD10⁺, CD105⁺, CD200⁺ placental cells are additionally CD90⁺ and CD45⁻, as detected by flow cytometry, *i.e.,* the cells are CD34⁻, CD10⁺, CD45⁻, CD90⁺, CD105⁺ and CD200⁺. In another specific embodiment, said CD34⁻, CD10⁺, CD45⁻, CD90⁺, CD105⁺, CD200⁺ cells are additionally CD80⁻ and CD86⁻.

In certain embodiments, said placental cells are CD34⁻, CD10⁺, CD105⁺ and CD200⁺, and one or more of CD38⁻, CD45⁻, CD80⁻, CD86⁻, CD133⁻, HLA-DR,DP,DQ⁻, SSEA3⁻, SSEA4⁻, CD29⁺, CD44⁺, CD73⁺, CD90⁺, CD105⁺, HLA-A,B,C⁺, PDL1⁺, ABC-p⁺, and/or OCT-4⁺, as detected by flow cytometry. In other embodiments, any of the CD34⁻, CD10⁺, CD105⁺ cells described above are additionally one or more of CD29⁺, CD38⁻, CD44⁺, CD54⁺, SH3⁺ or SH4⁺. In another specific embodiment, the cells are additionally CD44⁺. In another specific embodiment of any of the isolated CD34⁻, CD10⁺, CD105⁺ placental cells above, the cells are additionally one or more of CD117⁻, CD133⁻, KDR⁻ (VEGFR2⁻), HLA-A,B,C⁺, HLA-DP,DQ,DR⁻, or Programmed Death-1 Ligand (PDL1)⁺, or any combination thereof.

In another embodiment, the CD34―, CD10+, CD105+ cells are additionally one or more of CD13+, CD29+, CD33+, CD38―, CD44+, CD45―, CD54+, CD62E―, CD62L―, CD62P―, SH3+ (CD73+), SH4+ (CD73+), CD80-, CD86-, CD90+, SH2+ (CD105+), CD106/VCAM+, CD117―, CD144/VE-cadherinlow, CD184/CXCR4―, CD200+, CD133―, OCT-4+, SSEA3―, SSEA4-, ABC-p+, KDR- (VEGFR2-), HLA-A,B,C+, HLA-DP,DQ,DR-, HLA-G-, or Programmed Death-1 Ligand (PDL1)+, or any combination thereof. In a other embodiment, the CD34-, CD10+, CD105+ cells are additionally CD13+, CD29+, CD33+, CD38-, CD44+, CD45-, CD54/ICAM+, CD62E-, CD62L-, CD62P-, SH3+ (CD73+), SH4+ (CD73+), CD80―, CD86-, CD90+, SH2+ (CD105+), CD106/VCAM+, CD117―, CD144/VE-cadherinlow, CD184/CXCR4―, CD200+, CD133―, OCT-4+, SSEA3―, SSEA4―, ABC-p+, KDR― (VEGFR2― ), HLA-A,B,C+, HLA-DP,DQ,DR―, HLA-G-, and Programmed Death-1 Ligand (PDL1)+.

In another specific embodiment, any of the placental cells described herein are additionally ABC-p+, as detected by flow cytometry, or OCT-4+ (POU5F1+), as determined by RT-PCR, wherein ABC-p is a placenta-specific ABC transporter protein (also known as breast cancer resistance protein (BCRP) and as mitoxantrone resistance protein (MXR)), and OCT-4 is the Octamer-4 protein (POU5F1). In another specific embodiment, any of the placental cells described herein are additionally SSEA3- or SSEA4-, as determined by flow cytometry, wherein SSEA3 is Stage Specific Embryonic Antigen 3, and SSEA4 is Stage Specific Embryonic Antigen 4. In another specific embodiment, any of the placental cells described herein are additionally SSEA3- and SSEA4-.

In another specific embodiment, any of the placental cells described herein are additionally one or more of MHC-I+ (e.g., HLA-A,B,C+), MHC-II― (e.g., HLA-DP,DQ,DR―) or HLA-G-. In another specific embodiment, any of the placental cells described herein are additionally one or more of MHC-I+ (e.g., HLA-A,B,C+), MHC-II― (e.g., HLA-DP,DQ,DR―) and HLA-G-.

Also provided herein are populations of the isolated placental cells, or populations of cells, e.g., populations of placental cells, comprising, e.g., that are enriched for, the isolated placental cells, that are useful in the methods and compositions disclosed herein. Preferred populations of cells comprising the isolated placental cells, wherein the populations of cells comprise, e.g., at least 10%, 15%, 20%, 25%, 30%, 35%, 40%, 45%, 50%, 55%, 60%, 65%, 70%, 75%, 80%, 85%, 90%, 95% or 98% isolated CD10+, CD105+ and CD34― placental cells; that is, at least 10%, 15%, 20%, 25%, 30%, 35%, 40%, 45%, 50%, 55%, 60%, 65%, 70%, 75%, 80%, 85%, 90%, 95% or 98% of cells in said population are isolated CD10+, CD105+ and CD34- placental cells. In a specific embodiment, the isolated CD34―, CD10+, CD105+ placental cells are additionally CD200+. In another specific embodiment, the isolated CD34-, CD10+, CD105+, CD200+ placental cells are additionally CD90+ or CD45―, as detected by flow cytometry. In another specific embodiment, the isolated CD34―, CD10+, CD105+, CD200+ placental cells are additionally CD90+ and CD45-, as detected by flow cytometry. In another specific embodiment, any of the isolated CD34―, CD10+, CD105+ placental cells described above are additionally one or more of CD29+, CD38―, CD44+, CD54+, SH3+ or SH4+. In another specific embodiment, the isolated CD34―, CD10+, CD105+ placental cells, or isolated CD34―, CD10+, CD105+, CD200+ placental cells, are additionally CD44+. In a specific embodiment of any of the populations of cells comprising isolated CD34―, CD10+, CD105+ placental cells above, the isolated placental cells are additionally one or more of CD13+, CD29+, CD33+, CD38―, CD44+, CD45―, CD54+, CD62E―, CD62L―, CD62P―, SH3+ (CD73+), SH4+ (CD73+), CD80―, CD86―, CD90+, SH2+ (CD105+), CD106/VCAM+, CD117―, CD144/VE-cadherinlow, CD184/CXCR4-, CD200+, CD133-, OCT-4+, SSEA3-, SSEA4-, ABC-p+, KDR― (VEGFR2―), HLA-A,B,C+, HLA-DP,DQ,DR―, HLA-G―, or Programmed Death-1 Ligand (PDL1)+, or any combination thereof. In another specific embodiment, the CD34-, CD10+, CD105+ cells are additionally CD13+, CD29+, CD33+, CD38-, CD44+, CD45-, CD54/ICAM+, CD62E―, CD62L―, CD62P―, SH3+ (CD73+), SH4+ (CD73+), CD80―, CD86―, CD90+, SH2+ (CD105+), CD106/VCAM+, CD117―, CD144/VE-cadherinlow, CD184/CXCR4― , CD200+, CD133-, OCT-4+, SSEA3-, SSEA4-, ABC-p+, KDR- (VEGFR2-), HLA-A,B,C+, HLA-DP,DQ,DR―, HLA-G―, and Programmed Death-1 Ligand (PDL1)+.

In certain embodiments, the isolated placental cells useful in the methods and compositions described herein are one or more, or all, of CD10+, CD29+, CD34-, CD38-, CD44+, CD45―, CD54+, CD90+, SH2+, SH3+, SH4+, SSEA3―, SSEA4―, OCT-4+, and ABC-p+, wherein said isolated placental cells are obtained by physical and/or enzymatic disruption of placental tissue. In a specific embodiment, the isolated placental cells are OCT-4+ and ABC-p+. In another specific embodiment, the isolated placental cells are OCT-4+ and CD34-, wherein said isolated placental cells have at least one of the following characteristics: CD10+, CD29+, CD44+, CD45―, CD54+, CD90+, SH3+, SH4+, SSEA3―, and SSEA4―. In another specific embodiment, the isolated placental cells are OCT-4+, CD34―, CD10+, CD29+, CD44+, CD45―, CD54+, CD90+, SH3+, SH4+, SSEA3-, and SSEA4-. In another embodiment, the isolated placental cells are OCT-4+, CD34-, SSEA3-, and SSEA4-. In another specific embodiment, the isolated placental cells are OCT-4+ and CD34-, and is either SH2+ or SH3+. In another specific embodiment, the isolated placental cells are OCT-4+, CD34―, SH2+, and SH3+. In another specific embodiment, the isolated placental cells are OCT-4+, CD34―, SSEA3―, and SSEA4―, and are either SH2+ or SH3+. In another specific embodiment, the isolated placental cells are OCT-4+ and CD34-, and either SH2+ or SH3+, and is at least one of CD10+, CD29+, CD44+, CD45-, CD54+, CD90+, SSEA3―, or SSEA4―. In another specific embodiment, the isolated placental cells are OCT-4+, CD34―, CD10+, CD29+, CD44+, CD45―, CD54+, CD90+, SSEA3― , and SSEA4-, and either SH2+ or SH3+.

In another embodiment, the isolated placental cells useful in the methods and compositions disclosed herein are SH2+, SH3+, SH4+ and OCT-4+. In another specific embodiment, the isolated placental cells are CD10+, CD29+, CD44+, CD54+, CD90+, CD34―, CD45―, SSEA3―, or SSEA4―. In another embodiment, the isolated placental cells are SH2+, SH3+, SH4+, SSEA3― and SSEA4―. In another specific embodiment, the isolated placental cells are SH2+, SH3+, SH4+, SSEA3- and SSEA4―-, CD10+, CD29+, CD44+, CD54+, CD90+, OCT-4+, CD34- or CD45-.

In another embodiment, the isolated placental cells useful in the methods and compositions disclosed herein are CD10+, CD29+, CD34―, CD44+, CD45―, CD54+, CD90+, SH2+, SH3+, and SH4+; wherein said isolated placental cells are additionally one or more of OCT-4+, SSEA3- or SSEA4-.

In certain embodiments, isolated placental cells useful in the methods and compositions disclosed herein are CD200+ or HLA-G-. In a specific embodiment, the isolated placental cells are CD200+ and HLA-G-. In another specific embodiment, the isolated placental cells are additionally CD73+ and CD105+. In another specific embodiment, the isolated placental cells are additionally CD34-, CD38- or CD45-. In another specific embodiment, the isolated placental cells are additionally CD34-, CD38- and CD45-. In another specific embodiment, said stem cells are CD34-, CD38-, CD45-, CD73+ and CD105+. In another specific embodiment, said isolated CD200+ or HLA-G- placental cells facilitate the formation of embryoid-like bodies in a population of placental cells comprising the isolated placental cells, under conditions that allow the formation of embryoid-like bodies. In another specific embodiment, the isolated placental cells are isolated away from placental cells that are not stem or multipotent cells. In another specific embodiment, said isolated placental cells are isolated away from placental cells that do not display these markers.

In another embodiment, a cell population useful in the methods and compositions described herein is a population of cells comprising, e.g., that is enriched for, CD200+, HLA-G-stem cells. In a specific embodiment, said population is a population of placental cells. In various embodiments, at least about 10%, at least about 20%, at least about 30%, at least about 40%, at least about 50%, or at least about 60% of cells in said cell population are isolated CD200+, HLA-G― placental cells. Preferably, at least about 70% of cells in said cell population are isolated CD200+, HLA-G― placental cells. More preferably, at least about 90%, 95%, or 99% of said cells are isolated CD200+, HLA-G― placental cells. In a specific embodiment of the cell populations, said isolated CD200+, HLA-G― placental cells are also CD73+ and CD105+. In another specific embodiment, said isolated CD200+, HLA-G― placental cells are also CD34-, CD38- or CD45-. In another specific embodiment, said isolated CD200+, HLA-G― placental cells are also CD34-, CD38-, CD45-, CD73+ and CD105+. In another embodiment, said cell population produces one or more embryoid-like bodies when cultured under conditions that allow the formation of embryoid-like bodies. In another specific embodiment, said cell population is isolated away from placental cells that are not stem cells. In another specific embodiment, said isolated CD200+, HLA-G― placental cells are isolated away from placental cells that do not display these markers.

In another embodiment, the isolated placental cells useful in the methods and compositions described herein are CD73+, CD105+, and CD200+. In another specific embodiment, the isolated placental cells are HLA-G-. In another specific embodiment, the isolated placental cells are CD34-, CD38- or CD45-. In another specific embodiment, the isolated placental cells are CD34-, CD38- and CD45-. In another specific embodiment, the isolated placental cells are CD34-, CD38-, CD45-, and HLA-G-. In another specific embodiment, the isolated CD73+, CD105+, and CD200+ placental cells facilitate the formation of one or more embryoid-like bodies in a population of placental cells comprising the isolated placental cells, when the population is cultured under conditions that allow the formation of embryoid-like bodies. In another specific embodiment, the isolated placental cells are isolated away from placental cells that are not the isolated placental cells. In another specific embodiment, the isolated placental cells are isolated away from placental cells that do not display these markers.

In another embodiment, a cell population useful in the methods and compositions described herein is a population of cells comprising, e.g., that is enriched for, isolated CD73+, CD105+, CD200+ placental cells. In various embodiments, at least about 10%, at least about 20%, at least about 30%, at least about 40%, at least about 50%, or at least about 60% of cells in said cell population are isolated CD73+, CD105+, CD200+ placental cells. In another embodiment, at least about 70% of said cells in said population of cells are isolated CD73+, CD105+, CD200+ placental cells. In another embodiment, at least about 90%, 95% or 99% of cells in said population of cells are isolated CD73+, CD105+, CD200+ placental cells. In a specific embodiment of said populations, the isolated placental cells are HLA-G-. In another specific embodiment, the isolated placental cells are additionally CD34-, CD38- or CD45-. In another specific embodiment, the isolated placental cells are additionally CD34-, CD38- and CD45-. In another specific embodiment, the isolated placental cells are additionally CD34-, CD38-, CD45-, and HLA-G-. In another specific embodiment, said population of cells produces one or more embryoid-like bodies when cultured under conditions that allow the formation of embryoid-like bodies. In another specific embodiment, said population of placental cells is isolated away from placental cells that are not stem cells. In another specific embodiment, said population of placental cells is isolated away from placental cells that do not display these characteristics.

In certain other embodiments, the isolated placental cells are one or more of CD10+, CD29+, CD34-, CD38-, CD44+, CD45-, CD54+, CD90+, SH2+, SH3+, SH4+, SSEA3-, SSEA4-, OCT-4+, HLA-G― or ABC-p+. In a specific embodiment, the isolated placental cells are CD10+, CD29+, CD34-, CD38-, CD44+, CD45-, CD54+, CD90+, SH2+, SH3+, SH4+, SSEA3-, SSEA4-, and OCT-4+. In another specific embodiment, the isolated placental cells are CD10+, CD29+, CD34-, CD38-, CD45-, CD54+, SH2+, SH3+, and SH4+. In another specific embodiment, the isolated placental cells are CD10+, CD29+, CD34-, CD38-, CD45-, CD54+, SH2+, SH3+, SH4+ and OCT-4+. In another specific embodiment, the isolated placental cells are CD10+, CD29+, CD34-, CD38-, CD44+, CD45-, CD54+, CD90+, HLA-G-, SH2+, SH3+, SH4+. In another specific embodiment, the isolated placental cells are OCT-4+ and ABC-p+. In another specific embodiment, the isolated placental cells are SH2+, SH3+, SH4+ and OCT-4+. In another embodiment, the isolated placental cells are OCT-4+, CD34-, SSEA3-, and SSEA4-. In a specific embodiment, said isolated OCT-4+, CD34-, SSEA3-, and SSEA4- placental cells are additionally CD10+, CD29+, CD34-, CD44+, CD45-, CD54+, CD90+, SH2+, SH3+, and SH4+. In another embodiment, the isolated placental cells are OCT-4+ and CD34-, and either SH3+ or SH4+. In another embodiment, the isolated placental cells are CD34- and either CD10+, CD29+, CD44+, CD54+, CD90+, or OCT-4+.

In another embodiment, the isolated placental cells useful in the methods and compositions described herein are CD200+ and OCT-4+. In a specific embodiment, the isolated placental cells are CD73+ and CD105+. In another specific embodiment, said isolated placental cells are HLA-G-. In another specific embodiment, said isolated CD200+, OCT-4+ placental cells are CD34-, CD38- or CD45-. In another specific embodiment, said isolated CD200+, OCT-4+ placental cells are CD34-, CD38- and CD45-. In another specific embodiment, said isolated CD200+, OCT-4+ placental cells are CD34-, CD38-, CD45-, CD73+, CD105+ and HLA-G―. In another specific embodiment, the isolated CD200+, OCT-4+ placental cells facilitate the production of one or more embryoid-like bodies by a population of placental cells that comprises the isolated cells, when the population is cultured under conditions that allow the formation of embryoid-like bodies. In another specific embodiment, said isolated CD200+, OCT-4+ placental cells are isolated away from placental cells that are not stem cells. In another specific embodiment, said isolated CD200+, OCT-4+ placental cells are isolated away from placental cells that do not display these characteristics.

In another embodiment, a cell population useful in the methods and compositions described herein is a population of cells comprising, e.g., that is enriched for, CD200+, OCT-4+ placental cells. In various embodiments, at least about 10%, at least about 20%, at least about 30%, at least about 40%, at least about 50%, or at least about 60% of cells in said cell population are isolated CD200+, OCT-4+ placental cells. In another embodiment, at least about 70% of said cells are said isolated CD200+, OCT-4+ placental cells. In another embodiment, at least about 80%, 90%, 95%, or 99% of cells in said cell population are said isolated CD200+, OCT-4+ placental cells. In a specific embodiment of the isolated populations, said isolated CD200+, OCT-4+ placental cells are additionally CD73+ and CD105+. In another specific embodiment, said isolated CD200+, OCT-4+ placental cells are additionally HLA-G-. In another specific embodiment, said isolated CD200+, OCT-4+ placental cells are additionally CD34-, CD38- and CD45-. In another specific embodiment, said isolated CD200+, OCT-4+ placental cells are additionally CD34-, CD38-, CD45-, CD73+, CD105+ and HLA-G-. In another specific embodiment, the cell population produces one or more embryoid-like bodies when cultured under conditions that allow the formation of embryoid-like bodies. In another specific embodiment, said cell population is isolated away from placental cells that are not isolated CD200+, OCT-4+ placental cells. In another specific embodiment, said cell population is isolated away from placental cells that do not display these markers.

In another embodiment, the isolated placental cells useful in the methods and compositions described herein are CD73+, CD105+ and HLA-G-. In another specific embodiment, the isolated CD73+, CD105+ and HLA-G― placental cells are additionally CD34-, CD38- or CD45-. In another specific embodiment, the isolated CD73+, CD105+, HLA-G― placental cells are additionally CD34-, CD38- and CD45-. In another specific embodiment, the isolated CD73+, CD105+, HLA-G- placental cells are additionally OCT-4+. In another specific embodiment, the isolated CD73+, CD105+, HLA-G― placental cells are additionally CD200+. In another specific embodiment, the isolated CD73+, CD105+, HLA-G― placental cells are additionally CD34-, CD38-, CD45-, OCT-4+ and CD200+. In another specific embodiment, the isolated CD73+, CD105+, HLA-G― placental cells facilitate the formation of embryoid-like bodies in a population of placental cells comprising said cells, when the population is cultured under conditions that allow the formation of embryoid-like bodies. In another specific embodiment, said the isolated CD73+, CD105+, HLA-G― placental cells are isolated away from placental cells that are not the isolated CD73+, CD105+, HLA-G― placental cells. In another specific embodiment, said the isolated CD73+, CD105+, HLA-G― placental cells are isolated away from placental cells that do not display these markers.

In another embodiment, a cell population useful in the methods and compositions described herein is a population of cells comprising, e.g., that is enriched for, isolated CD73+, CD105+ and HLA-G- placental cells. In various embodiments, at least about 10%, at least about 20%, at least about 30%, at least about 40%, at least about 50%, or at least about 60% of cells in said population of cells are isolated CD73+, CD105+, HLA-G- placental cells. In another embodiment, at least about 70% of cells in said population of cells are isolated CD73+, CD105+, HLA-G― placental cells. In another embodiment, at least about 90%, 95% or 99% of cells in said population of cells are isolated CD73+, CD105+, HLA-G- placental cells. In a specific embodiment of the above populations, said isolated CD73+, CD105+, HLA-G― placental cells are additionally CD34-, CD38- or CD45-. In another specific embodiment, said isolated CD73+, CD105+, HLA-G― placental cells are additionally CD34-, CD38- and CD45-. In another specific embodiment, said isolated CD73+, CD105+, HLA-G- placental cells are additionally OCT-4+. In another specific embodiment, said isolated CD73+, CD105+, HLA-G― placental cells are additionally CD200+. In another specific embodiment, said isolated CD73+, CD105+, HLA-G― placental cells are additionally CD34-, CD38-, CD45-, OCT-4+ and CD200+. In another specific embodiment, said cell population is isolated away from placental cells that are not CD73+, CD105+, HLA-G― placental cells. In another specific embodiment, said cell population is isolated away from placental cells that do not display these markers.

In another embodiment, the isolated placental cells useful in the methods and compositions described herein are CD73+ and CD105+ and facilitate the formation of one or more embryoid-like bodies in a population of isolated placental cells comprising said CD73+, CD105+ cells when said population is cultured under conditions that allow formation of embryoid-like bodies. In another specific embodiment, said isolated CD73+, CD105+ placental cells are additionally CD34-, CD38- or CD45-. In another specific embodiment, said isolated CD73+, CD105+ placental cells are additionally CD34-, CD38- and CD45-. In another specific embodiment, said isolated CD73+, CD105+ placental cells are additionally OCT-4+. In another specific embodiment, said isolated CD73+, CD105+ placental cells are additionally OCT-4+, CD34-, CD38- and CD45-. In another specific embodiment, said isolated CD73+, CD105+ placental cells are isolated away from placental cells that are not said cells. In another specific embodiment, said isolated CD73+, CD105+ placental cells are isolated away from placental cells that do not display these characteristics.

In another embodiment, a cell population useful in the methods and compositions described herein is a population of cells comprising, e.g., that is enriched for, isolated placental cells that are CD73+, CD105+ and facilitate the formation of one or more embryoid-like bodies in a population of isolated placental cells comprising said cells when said population is cultured under conditions that allow formation of embryoid-like bodies. In various embodiments, at least about 10%, at least about 20%, at least about 30%, at least about 40%, at least about 50%, or at least about 60% of cells in said population of cells are said isolated CD73+, CD105+ placental cells. In another embodiment, at least about 70% of cells in said population of cells are said isolated CD73+, CD105+ placental cells. In another embodiment, at least about 90%, 95% or 99% of cells in said population of cells are said isolated CD73+, CD105+ placental cells. In a specific embodiment of the above populations, said isolated CD73+, CD105+ placental cells are additionally CD34-, CD38- or CD45-. In another specific embodiment, said isolated CD73+, CD105+ placental cells are additionally CD34-, CD38- and CD45-. In another specific embodiment, said isolated CD73+, CD105+ placental cells are additionally OCT-4+. In another specific embodiment, said isolated CD73+, CD105+ placental cells are additionally CD200+. In another specific embodiment, said isolated CD73+, CD105+ placental cells are additionally CD34-, CD38-, CD45-, OCT-4+ and CD200+. In another specific embodiment, said cell population is isolated away from placental cells that are not said isolated CD73+, CD105+ placental cells. In another specific embodiment, said cell population is isolated away from placental cells that do not display these markers.

In another embodiment, the isolated placental cells useful in the methods and compositions described herein are OCT-4+ and facilitate formation of one or more embryoid-like bodies in a population of isolated placental cells comprising said cells when cultured under conditions that allow formation of embryoid-like bodies. In a specific embodiment, said isolated OCT-4+ placental cells are additionally CD73+ and CD105+. In another specific embodiment, said isolated OCT-4+ placental cells are additionally CD34-, CD38-, or CD45-. In another specific embodiment, said isolated OCT-4+ placental cells are additionally CD200+. In another specific embodiment, said isolated OCT-4+ placental cells are additionally CD73+, CD105+, CD200+, CD34-, CD38-, and CD45-. In another specific embodiment, said isolated OCT-4+ placental cells are isolated away from placental cells that are not OCT-4+ placental cells. In another specific embodiment, said isolated OCT-4+ placental cells are isolated away from placental cells that do not display these characteristics.

In another embodiment, a cell population useful in the methods and compositions described herein is a population of cells comprising, e.g., that is enriched for, isolated placental cells that are OCT-4+ and facilitate the formation of one or more embryoid-like bodies in a population of isolated placental cells comprising said cells when said population is cultured under conditions that allow formation of embryoid-like bodies. In various embodiments, at least about 10%, at least about 20%, at least about 30%, at least about 40%, at least about 50%, or at least about 60% of cells in said population of cells are said isolated OCT-4+ placental cells. In another embodiment, at least about 70% of cells in said population of cells are said isolated OCT-4+ placental cells. In another embodiment, at least about 80%, 90%, 95% or 99% of cells in said population of cells are said isolated OCT-4+ placental cells. In a specific embodiment of the above populations, said isolated OCT-4+ placental cells are additionally CD34-, CD38- or CD45-. In another specific embodiment, said isolated OCT-4+ placental cells are additionally CD34-, CD38- and CD45-. In another specific embodiment, said isolated OCT-4+ placental cells are additionally CD73+ and CD105+. In another specific embodiment, said isolated OCT-4+ placental cells are additionally CD200+. In another specific embodiment, said isolated OCT-4+ placental cells are additionally CD73+, CD105+, CD200+, CD34-, CD38-, and CD45-. In another specific embodiment, said cell population is isolated away from placental cells that are not said cells. In another specific embodiment, said cell population is isolated away from placental cells that do not display these markers.

In another embodiment, the isolated placental cells useful in the methods and compositions described herein are isolated HLA-A,B,C+, CD45-, CD133- and CD34- placental cells. In another embodiment, a cell population useful in the methods and compositions described herein is a population of cells comprising isolated placental cells, wherein at least about 70%, at least about 80%, at least about 90%, at least about 95% or at least about 99% of cells in said isolated population of cells are isolated HLA-A,B,C+, CD45-, CD133- and CD34-placental cells. In a specific embodiment, said isolated placental cell or population of isolated placental cells is isolated away from placental cells that are not HLA-A,B,C+, CD45-, CD133― and CD34- placental cells. In another specific embodiment, said isolated placental cells are non-maternal in origin. In another specific embodiment, said isolated population of placental cells are substantially free of maternal components; e.g., at least about 40%, 45%, 5-0%, 55%, 60%, 65%, 70%, 75%, 90%, 85%, 90%, 95%, 98% or 99% of said cells in said isolated population of placental cells are non-maternal in origin.

In another embodiment, the isolated placental cells useful in the methods and compositions described herein are isolated CD10+, CD13+, CD33+, CD45-, CD117- and CD133- placental cells. In another embodiment, a cell population useful in the methods and compositions described herein is a population of cells comprising isolated placental cells, wherein at least about 70%, at least about 80%, at least about 90%, at least about 95% or at least about 99% of cells in said population of cells are isolated CD10+, CD13+, CD33+, CD45-, CD117- and CD133- placental cells. In a specific embodiment, said isolated placental cells or population of isolated placental cells is isolated away from placental cells that are not said isolated placental cells. In another specific embodiment, said isolated CD10+, CD13+, CD33+, CD45-, CD117- and CD133- placental cells are non-maternal in origin, i.e., have the fetal genotype. In another specific embodiment, at least about 40%, 45%, 50%, 55%, 60%, 65%, 70%, 75%, 90%, 85%, 90%, 95%, 98% or 99% of said cells in said isolated population of placental cells, are non-maternal in origin. In another specific embodiment, said isolated placental cells or population of isolated placental cells are isolated away from placental cells that do not display these characteristics.

In another embodiment, the isolated placental cells useful in the methods and compositions described herein are isolated CD10―, CD33-, CD44+, CD45-, and CD117-placental cells. In another embodiment, a cell population useful for the in the methods and compositions described herein is a population of cells comprising, e.g., enriched for, isolated placental cells, wherein at least about 70%, at least about 80%, at least about 90%, at least about 95% or at least about 99% of cells in said population of cells are isolated CD10―, CD33-, CD44+, CD45-, and CD117- placental cells. In a specific embodiment, said isolated placental cell or population of isolated placental cells is isolated away from placental cells that are not said cells. In another specific embodiment, said isolated placental cells are non-maternal in origin. In another specific embodiment, at least about 40%, 45%, 50%, 55%, 60%, 65%, 70%, 75%, 90%, 85%, 90%, 95%, 98% or 99% of said cells in said cell population are non-maternal in origin. In another specific embodiment, said isolated placental cell or population of isolated placental cells is isolated away from placental cells that do not display these markers.

In another embodiment, the isolated placental cells useful in the methods and compositions described herein are isolated CD10―, CD13-, CD33-, CD45-, and CD117-placental cells. In another embodiment, a cell population useful for in the methods and compositions described herein is a population of cells comprising, e.g., enriched for, isolated CD10-, CD13-, CD33-, CD45-, and CD117- placental cells, wherein at least about 70%, at least about 80%, at least about 90%, at least about 95% or at least about 99% of cells in said population are CD10―, CD13-, CD33-, CD45-, and CD117― placental cells. In a specific embodiment, said isolated placental cells or population of isolated placental cells are isolated away from placental cells that are not said cells. In another specific embodiment, said isolated placental cells are non-maternal in origin. In another specific embodiment, at least about 40%, 45%, 50%, 55%, 60%, 65%, 70%, 75%, 90%, 85%, 90%, 95%, 98% or 99% of said cells in said cell population are non-maternal in origin. In another specific embodiment, said isolated placental cells or population of isolated placental cells is isolated away from placental cells that do not display these characteristics.

In another embodiment, the isolated placental cells useful in the methods and compositions described herein are HLA A,B,C+, CD45-, CD34-, and CD133-, and are additionally CD10+, CD13+, CD38+, CD44+, CD90+, CD105+, CD200+ and/or HLA-G-, and/or negative for CD117. In another embodiment, a cell population useful in the methods described herein is a population of cells comprising isolated placental cells, wherein at least about 20%, 25%, 30%, 35%, 40%, 45%, 50%, 55%, 60%, 65%, 70%, 75%, 80%, 85%, 90%, 95%, 98% or about 99% of the cells in said population are isolated placental cells that are HLA A,B,C-, CD45-, CD34-, CD133-, and that are additionally positive for CD10, CD13, CD38, CD44, CD90, CD105, CD200, and/or negative for CD117 and/or HLA-G. In a specific embodiment, said isolated placental cells or population of isolated placental cells are isolated away from placental cells that are not said cells. In another specific embodiment, said isolated placental cells are non-maternal in origin. In another specific embodiment, at least about 40%, 45%, 50%, 55%, 60%, 65%, 70%, 75%, 90%, 85%, 90%, 95%, 98% or 99% of said cells in said cell population are non-maternal in origin. In another specific embodiment, said isolated placental cells or population of isolated placental cells are isolated away from placental cells that do not display these markers.

In another embodiment, the isolated placental cells useful in the methods and compositions described herein are isolated placental cells that are CD200+ and CD10+, as determined by antibody binding, and CD117-, as determined by both antibody binding and RT-PCR. In another embodiment, the isolated placental cells useful in the methods and compositions described herein are isolated placental cells, e.g., placental stem cells or placental multipotent cells, that are CD10+, CD29-, CD54+, CD200+, HLA-G-, MHC class I+ and β-2-microglobulin+. In another embodiment, isolated placental cells useful in the methods and compositions described herein are placental cells wherein the expression of at least one cellular marker is at least two-fold higher than for a mesenchymal stem cell (e.g., a bone marrow-derived mesenchymal stem cell). In another specific embodiment, said isolated placental cells are non-maternal in origin. In another specific embodiment, at least about 40%, 45%, 50%, 55%, 60%, 65%, 70%, 75%, 90%, 85%, 90%, 95%, 98% or 99% of said cells in said cell population are non-maternal in origin.

In another embodiment, the isolated placental cells useful in the methods and compositions described herein are isolated placental cells, e.g., placental stem cells or placental multipotent cells, that are one or more of CD10+, CD29+, CD44+, CD45-, CD54/ICAM+, CD62E-, CD62L-, CD62P-, CD80-, CD86-, CD103-, CD104-, CD105+, CD106/VCAM+, CD144/VE-cadherinlow, CD184/CXCR4―, β2-microglobulinlow, MHC-Ilow, MHC-II-, HLA-Glow, and/or PDL1low. In a specific embodiment, the isolated placental cells are at least CD29+ and CD54+. In another specific embodiment, the isolated placental cells are at least CD44+ and CD106+. In another specific embodiment, the isolated placental cells are at least CD29+.

In another embodiment, a cell population useful in the methods and compositions described herein comprises isolated placental cells, and at least 50%, 60%, 70%, 80%, 90%, 95%, 98% or 99% of the cells in said cell population are isolated placental cells that are one or more of CD10+, CD29+, CD44+, CD45-, CD54/ICAM+, CD62-E-, CD62-L-, CD62-P-, CD80-, CD86-, CD103-, CD104-, CD105+, CD106/VCAM+, CD144/VE-cadherindim, CD184/CXCR4―, β2-microglobulindim, HLA-Idim, HLA-II-, HLA-Gdim, and/or PDL1dim. In another specific embodiment, at least 50%, 60%, 70%, 80%, 90%, 95%, 98% or 99% of cells in said cell population are CD10+, CD29+, CD44+, CD45-, CD54/ICAM+, CD62-E-, CD62-L―, CD62-P-, CD80-, CD86-, CD103-, CD104-, CD105+, CD106/VCAM+, CD144/VE-cadherindim, CD184/CXCR4-, β2-microglobulindim, MHC-Idim, MHC-II-, HLA-Gdim, and PDL1dim.

In another embodiment, the isolated placental cells useful in the methods and compositions described herein are isolated placental cells that are one or more, or all, of CD10+, CD29+, CD34-, CD38-, CD44+, CD45-, CD54+, CD90+, SH2+, SH3+, SH4+, SSEA3―, SSEA4-, OCT-4+, and ABC-p+, where ABC-p is a placenta-specific ABC transporter protein (also known as breast cancer resistance protein (BCRP) and as mitoxantrone resistance protein (MXR)), wherein said isolated placental cells are obtained by perfusion of a mammalian, e.g., human, placenta that has been drained of cord blood and perfused to remove residual blood.

In another specific embodiment of any of the above characteristics, expression of the cellular marker (e.g., cluster of differentiation or immunogenic marker) is determined by flow cytometry; in another specific embodiment, expression of the marker is determined by RT-PCR.

Gene profiling confirms that isolated placental cells, and populations of isolated placental cells, are distinguishable from other cells, e.g., mesenchymal stem cells, e.g., bone marrow-derived mesenchymal stem cells. The isolated placental cells described herein can be distinguished from, e.g., mesenchymal stem cells on the basis of the expression of one or more genes, the expression of which is significantly higher in the isolated placental cells, or in certain isolated umbilical cord stem cells, in comparison to bone marrow-derived mesenchymal stem cells. In particular, the isolated placental cells, useful in the methods of treatment provided herein, can be distinguished from mesenchymal stem cells on the basis of the expression of one or more genes, the expression of which is significantly higher (that is, at least twofold higher) in the isolated placental cells than in an equivalent number of bone marrow-derived mesenchymal stem cells, wherein the one or more genes are ACTG2, ADARB 1, AMIGO2, ARTS-1, B4GALT6, BCHE, C11orf9, CD200, COL4A1, COL4A2, CPA4, DMD, DSC3, DSG2, ELOVL2, F2RL1, FLJ10781, GATA6, GPR126, GPRC5B, HLA-G, ICAM1, IER3, IGFBP7, ILIA, IL6, IL18, KRT18, KRT8, LIPG, LRAP, MATN2, MEST, NFE2L3, NUAK1, PCDH7, PDLIM3, PKP2, RTN1, SERPINB9, ST3GAL6, ST6GALNAC5, SLC12A8, TCF21, TGFB2, VTN, ZC3H12A, or a combination of any of the foregoing, when the cells are grown under equivalent conditions. See, e.g., U.S. Patent Application Publication No. 2007/0275362, the disclosure of which is incorporated herein by reference in its entirety. In certain specific embodiments, said expression of said one ore more genes is determined, e.g., by RT-PCR or microarray analysis, e.g, using a U133-A microarray (Affymetrix). In another specific embodiment, said isolated placental cells express said one or more genes when cultured for a number of population doublings, e.g., anywhere from about 3 to about 35 population doublings, in a medium comprising DMEM-LG (e.g., from Gibco); 2% fetal calf serum (e.g., from Hyclone Labs.); 1x insulin-transferrin-selenium (ITS); 1x linoleic acid-bovine serum albumin (LA-BSA); 10-9 M dexamethasone (e.g., from Sigma); 10-4 M ascorbic acid 2-phosphate (e.g., from Sigma); epidermal growth factor 10 ng/mL (e.g., from R&D Systems); and platelet-derived growth factor (PDGF-BB) 10 ng/mL (e.g., from R&D Systems). In another specific embodiment, the isolated placental cell-specific or isolated umbilical cord cell-specific gene is CD200.

Specific sequences for these genes can be found in GenBank at accession nos. NM_001615 (ACTG2), BC065545 (ADARB1), (NM_181847 (AMIGO2), AY358590 (ARTS-1), BC074884 (B4GALT6), BC008396 (BCHE), BC020196 (C11orf9), BC031103 (CD200), NM_001845 (COL4A1), NM_001846 (COL4A2), BC052289 (CPA4), BC094758 (DMD), AF293359 (DSC3), NM_001943 (DSG2), AF338241 (ELOVL2), AY336105 (F2RL1), NM_018215 (FLJ10781), AY416799 (GATA6), BC075798 (GPR126), NM_016235 (GPRC5B), AF340038 (ICAM1), BC000844 (IER3), BC066339 (IGFBP7), BC013142 (ILIA), BT019749 (IL6), BC007461 (IL18), (BC072017) KRT18, BC075839 (KRT8), BC060825 (LIPG), BC065240 (LRAP), BC010444 (MATN2), BC011908 (MEST), BC068455 (NFE2L3), NM_014840 (NUAK1), AB006755 (PCDH7), NM_014476 (PDLIM3), BC126199 (PKP-2), BC090862 (RTN1), BC002538 (SERPINB9), BC023312 (ST3GAL6), BC001201 (ST6GALNAC5), BC126160 or BC065328 (SLC12A8), BC025697 (TCF21), BC096235 (TGFB2), BC005046 (VTN), and BC005001 (ZC3H12A) as of March 2008.

In certain specific embodiments, said isolated placental cells express each of ACTG2, ADARB1, AMIGO2, ARTS-1, B4GALT6, BCHE, C11orf9, CD200, COL4A1, COL4A2, CPA4, DMD, DSC3, DSG2, ELOVL2, F2RL1, FLJ10781, GATA6, GPR126, GPRC5B, HLA-G, ICAM1, IER3, IGFBP7, ILIA, IL6, IL18, KRT18, KRT8, LIPG, LRAP, MATN2, MEST, NFE2L3, NUAK1, PCDH7, PDLIM3, PKP2, RTN1, SERPINB9, ST3GAL6, ST6GALNAC5, SLC12A8, TCF21, TGFB2, VTN, and ZC3H12A at a detectably higher level than an equivalent number of bone marrow-derived mesenchymal stem cells, when the cells are grown under equivalent conditions.

In specific embodiments, the placental cells express CD200 and ARTS1 (aminopeptidase regulator of type 1 tumor necrosis factor); ARTS-1 and LRAP (leukocyte-derived arginine aminopeptidase); IL6 (interleukin-6) and TGFB2 (transforming growth factor, beta 2); IL6 and KRT18 (keratin 18); IER3 (immediate early response 3), MEST (mesoderm specific transcript homolog) and TGFB2; CD200 and IER3; CD200 and IL6; CD200 and KRT18; CD200 and LRAP; CD200 and MEST; CD200 and NFE2L3 (nuclear factor (erythroid-derived 2)-like 3); or CD200 and TGFB2 at a detectably higher level than an equivalent number of bone marrow-derived mesenchymal stem cells (BM-MSCs) wherein said bone marrow-derived mesenchymal stem cells have undergone a number of passages in culture equivalent to the number of passages said isolated placental cells have undergone. In other specific embodiments, the placental cells express ARTS-1, CD200, IL6 and LRAP; ARTS-1, IL6, TGFB2, IER3, KRT18 and MEST; CD200, IER3, IL6, KRT18, LRAP, MEST, NFE2L3, and TGFB2; ARTS-1, CD200, IER3, IL6, KRT18, LRAP, MEST, NFE2L3, and TGFB2; or IER3, MEST and TGFB2 at a detectably higher level than an equivalent number of bone marrow-derived mesenchymal stem cells BM-MSCs, wherein said bone marrow-derived mesenchymal stem cells have undergone a number of passages in culture equivalent to the number of passages said isolated placental cells have undergone.

Expression of the above-referenced genes can be assessed by standard techniques. For example, probes based on the sequence of the gene(s) can be individually selected and constructed by conventional techniques. Expression of the genes can be assessed, e.g., on a microarray comprising probes to one or more of the genes, e.g., an Affymetrix GENECHIP^{®} Human Genome U133A 2.0 array, or an Affymetrix GENECHIP^{®} Human Genome U133 Plus 2.0 (Santa Clara, California). Expression of these genes can be assessed even if the sequence for a particular GenBank accession number is amended because probes specific for the amended sequence can readily be generated using well-known standard techniques.

The level of expression of these genes can be used to confirm the identity of a population of isolated placental cells, to identify a population of cells as comprising at least a plurality of isolated placental cells, or the like. Populations of isolated placental cells, the identity of which is confirmed, can be clonal, e.g., populations of isolated placental cells expanded from a single isolated placental cell, or a mixed population of stem cells, e.g., a population of cells comprising solely isolated placental cells that are expanded from multiple isolated placental cells, or a population of cells comprising isolated placental cells, as described herein, and at least one other type of cell.

The level of expression of these genes can be used to select populations of isolated placental cells. For example, a population of cells, e.g., clonally-expanded cells, may be selected if the expression of one or more of the genes listed above is significantly higher in a sample from the population of cells than in an equivalent population of mesenchymal stem cells. Such selecting can be of a population from a plurality of isolated placental cell populations, from a plurality of cell populations, the identity of which is not known, etc.

Isolated placental cells can be selected on the basis of the level of expression of one or more such genes as compared to the level of expression in said one or more genes in, e.g., a mesenchymal stem cell control, for example, the level of expression in said one or more genes in an equivalent number of bone marrow-derived mesenchymal stem cells. In one embodiment, the level of expression of said one or more genes in a sample comprising an equivalent number of mesenchymal stem cells is used as a control. In another embodiment, the control, for isolated placental cells tested under certain conditions, is a numeric value representing the level of expression of said one or more genes in mesenchymal stem cells under said conditions.

In certain embodiments, the placental cells (e.g., PDACs) useful in the methods provided herein, do not express CD34, as detected by immunolocalization, after exposure to 1 to 100 ng/mL VEGF for 4 to 21 days. In a specific embodiment, said placental adherent cells are adherent to tissue culture plastic. In another specific embodiment, said population of cells induce endothelial cells to form sprouts or tube-like structures when cultured in the presence of an angiogenic factor such as vascular endothelial growth factor (VEGF), epithelial growth factor (EGF), platelet derived growth factor (PDGF) or basic fibroblast growth factor (bFGF), e.g., on a substrate such as MATRIGEL^{™}.

In another aspect, the PDACs provided herein, a population of cells, e.g., a population of PDACs, or a population of cells wherein at least about 50%, 60%, 70%, 80%, 90%, 95% or 98% of cells in said isolated population of cells are PDACs, secrete one or more, or all, of VEGF, HGF, IL-8, MCP-3, FGF2, follistatin, G-CSF, EGF, ENA-78, GRO, IL-6, MCP-1, PDGF-BB, TIMP-2, uPAR, or galectin-1, e.g., into culture medium in which the cell, or cells, are grown. In another embodiment, the PDACs express increased levels of CD202b, IL-8 and/or VEGF under hypoxic conditions (e.g., less than about 5% 02) compared to normoxic conditions (e.g., about 20% or about 21% O2).

In another embodiment, any of the PDACS or populations of cells comprising PDACs described herein can cause the formation of sprouts or tube-like structures in a population of endothelial cells in contact with said placental derived adherent cells. In a specific embodiment, the PDACs are co-cultured with human endothelial cells, which form sprouts or tube-like structures, or support the formation of endothelial cell sprouts, e.g., when cultured in the presence of extracellular matrix proteins such as collagen type I and IV, and/or angiogenic factors such as vascular endothelial growth factor (VEGF), epithelial growth factor (EGF), platelet derived growth factor (PDGF) or basic fibroblast growth factor (bFGF), e.g., in or on a substrate such as placental collagen or MATRIGEL^{™} for at least 4 days. In another embodiment, any of the populations of cells comprising placental derived adherent cells, described herein, secrete angiogenic factors such as vascular endothelial growth factor (VEGF), hepatocyte growth factor (HGF), platelet derived growth factor (PDGF), basic fibroblast growth factor (bFGF), or Interleukin-8 (IL-8) and thereby can induce human endothelial cells to form sprouts or tube-like structures when cultured in the presence of extracellular matrix proteins such as collagen type I and IV e.g., in or on a substrate such as placental collagen or MATRIGEL^{™}.

In another embodiment, any of the above populations of cells comprising placental derived adherent cells (PDACs) secretes angiogenic factors. In specific embodiments, the population of cells secretes vascular endothelial growth factor (VEGF), hepatocyte growth factor (HGF), platelet derived growth factor (PDGF), basic fibroblast growth factor (bFGF), and/or interleukin-8 (IL-8). In other specific embodiments, the population of cells comprising PDACs secretes one or more angiogenic factors and thereby induces human endothelial cells to migrate in an in vitro wound healing assay. In other specific embodiments, the population of cells comprising placental derived adherent cells induces maturation, differentiation or proliferation of human endothelial cells, endothelial progenitors, myocytes or myoblasts.

The isolated placental cells described herein display the above characteristics (e.g., combinations of cell surface markers and/or gene expression profiles) in primary culture, or during proliferation in medium comprising, e.g., DMEM-LG (Gibco), 2% fetal calf serum (FCS) (Hyclone Laboratories), 1x insulin-transferrin-selenium (ITS), 1x lenolenic-acid-bovine-serum-albumin (LA-BSA), 10-9 M dexamethasone (Sigma), 10-4M ascorbic acid 2-phosphate (Sigma), epidermal growth factor (EGF)10ng/ml (R&D Systems), platelet derived-growth factor (PDGF-BB) 10ng/ml (R&D Systems), and 100U penicillin/1000U streptomycin.

In certain embodiments of any of the placental cells disclosed herein, the cells are human. In certain embodiments of any of the placental cells disclosed herein, the cellular marker characteristics or gene expression characteristics are human markers or human genes.

In another specific embodiment of said isolated placental cells or populations of cells comprising the isolated placental cells, said cells or population have been expanded, for example, passaged at least, about, or no more than, 1, 2, 3, 4, 5, 6, 7, 8, 9, 10, 11, 12, 13, 14, 15, 16, 17, 18, 19, or 20 times, or more, or proliferated for at least, about, or no more than, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 population doublings. In another specific embodiment of said isolated placental cells or populations of cells comprising the isolated placental cells, said cells or population are primary isolates. In another specific embodiment of the isolated placental cells, or populations of cells comprising isolated placental cells, that are disclosed herein, said isolated placental cells are fetal in origin (that is, have the fetal genotype).

In certain embodiments, said isolated placental cells do not differentiate during culturing in growth medium, i.e., medium formulated to promote proliferation, e.g., during proliferation in growth medium. In another specific embodiment, said isolated placental cells do not require a feeder layer in order to proliferate. In another specific embodiment, said isolated placental cells do not differentiate in culture in the absence of a feeder layer, solely because of the lack of a feeder cell layer.

In another embodiment, cells useful in the methods and compositions described herein are isolated placental cells, wherein a plurality of said isolated placental cells are positive for aldehyde dehydrogenase (ALDH), as assessed by an aldehyde dehydrogenase activity assay. Such assays are known in the art (see, e.g., Bostian and Betts, Biochem. J., 173, 787, (1978)). In a specific embodiment, said ALDH assay uses Aldefluor^{®} (Aldagen, Inc., Ashland, Oregon) as a marker of aldehyde dehydrogenase activity. In a specific embodiment, said plurality is between about 3% and about 25% of cells in said population of cells. In another embodiment, provided herein is a population of isolated umbilical cord cells, e.g., multipotent isolated umbilical cord cells, wherein a plurality of said isolated umbilical cord cells are positive for aldehyde dehydrogenase, as assessed by an aldehyde dehydrogenase activity assay that uses Aldefluor^{®} as an indicator of aldehyde dehydrogenase activity. In a specific embodiment, said plurality is between about 3% and about 25% of cells in said population of cells. In another embodiment, said population of isolated placental cells or isolated umbilical cord cells shows at least threefold, or at least five-fold, higher ALDH activity than a population of bone marrow-derived mesenchymal stem cells having about the same number of cells and cultured under the same conditions.

In certain embodiments of any of the populations of cells comprising the isolated placental cells described herein, the placental cells in said populations of cells are substantially free of cells having a maternal genotype; e.g., at least 40%, 45%, 50%, 55%, 60%, 65%, 70%, 75%, 80%, 85%, 90%, 95%, 98% or 99% of the placental cells in said population have a fetal genotype. In certain other embodiments of any of the populations of cells comprising the isolated placental cells described herein, the populations of cells comprising said placental cells are substantially free of cells having a maternal genotype; e.g., at least 40%, 45%, 50%, 55%, 60%, 65%, 70%, 75%, 80%, 85%, 90%, 95%, 98% or 99% of the cells in said population have a fetal genotype.

In a specific embodiment of any of the above isolated placental cells or cell populations of isolated placental cells, the karyotype of the cells, or at least about 95% or about 99% of the cells in said population, is normal. In another specific embodiment of any of the above placental cells or cell populations, the cells, or cells in the population of cells, are non-maternal in origin.

Isolated placental cells, or populations of isolated placental cells, bearing any of the above combinations of markers, can be combined in any ratio. Any two or more of the above isolated placental cell populations can be combined to form an isolated placental cell population. For example, an population of isolated placental cells can comprise a first population of isolated placental cells defined by one of the marker combinations described above, and a second population of isolated placental cells defined by another of the marker combinations described above, wherein said first and second populations are combined in a ratio of about 1:99, 2:98, 3:97, 4:96, 5:95, 10:90, 20:80, 30:70, 40:60, 50:50, 60:40, 70:30, 80:20, 90:10, 95:5, 96:4, 97:3, 98:2, or about 99:1. In like fashion, any three, four, five or more of the above-described isolated placental cells or isolated placental cells populations can be combined.

Isolated placental cells useful in the methods and compositions described herein can be obtained, e.g., by disruption of placental tissue, with or without enzymatic digestion (see Section 4.2.3) or perfusion (see Section 4.2.4). For example, populations of isolated placental cells can be produced according to a method comprising perfusing a mammalian placenta that has been drained of cord blood and perfused to remove residual blood; perfusing said placenta with a perfusion solution; and collecting said perfusion solution, wherein said perfusion solution after perfusion comprises a population of placental cells that comprises isolated placental cells; and isolating a plurality of said isolated placental cells from said population of cells. In a specific embodiment, the perfusion solution is passed through both the umbilical vein and umbilical arteries and collected after it exudes from the placenta. In another specific embodiment, the perfusion solution is passed through the umbilical vein and collected from the umbilical arteries, or passed through the umbilical arteries and collected from the umbilical vein.

In various embodiments, the isolated placental cells, contained within a population of cells obtained from perfusion of a placenta, are at least 50%, 60%, 70%, 80%, 90%, 95%, 99% or at least 99.5% of said population of placental cells. In another specific embodiment, the isolated placental cells collected by perfusion comprise fetal and maternal cells. In another specific embodiment, the isolated placental cells collected by perfusion are at least 50%, 60%, 70%, 80%, 90%, 95%, 99% or at least 99.5% fetal cells.

In another specific embodiment, provided herein is a composition comprising a population of the isolated placental cells, as described herein, collected by perfusion, wherein said composition comprises at least a portion of the perfusion solution used to collect the isolated placental cells.

Isolated populations of the isolated placental cells described herein can be produced by digesting placental tissue with a tissue-disrupting enzyme to obtain a population of placental cells comprising the cells, and isolating, or substantially isolating, a plurality of the placental cells from the remainder of said placental cells. The whole, or any part of, the placenta can be digested to obtain the isolated placental cells described herein. In specific embodiments, for example, said placental tissue can be a whole placenta, an amniotic membrane, chorion, a combination of amnion and chorion, or a combination of any of the foregoing. In other specific embodiment, the tissue-disrupting enzyme is trypsin or collagenase. In various embodiments, the isolated placental cells, contained within a population of cells obtained from digesting a placenta, are at least 50%, 60%, 70%, 80%, 90%, 95%, 99% or at least 99.5% of said population of placental cells.

The isolated populations of placental cells described above, and populations of isolated placental cells generally, can comprise about, at least, or no more than 1 × 10³, 3 × 10³, 5 × 10³, 1 × 10⁴, 3 × 10⁴, 5 × 10⁴, 1 × 10⁵, 1 3 × 10⁵, 5 × 10⁵, 1 × 10⁶, 3 × 10⁶, 5 × 10⁶, 1 × 10⁷, 3 × 10⁷, 5 × 10⁷, 1 × 10⁸, 3 × 10⁸, 5 × 10⁸, 1 × 10⁹, 5 × 10⁹, or 1 × 10¹⁰ isolated placental cells (e.g., as part of a pharmaceutical composition comprising placental stem cells) or between about 1 × 10³ to 3 × 10³, 3 × 10³ to 5 × 10³ 5 × 10³ to 1 × 10⁴, 1 × 10⁴ to 3 × 10⁴, 3 × 10⁴ to 5 × 10⁴, 5 × 10⁴ to 1 × 10⁵, 1 × 10⁵ to 3 × 10⁵, 3 × 10⁵ to 5 × 10⁵, 5 × 10⁵ to 1 × 10⁶, 1 × 10⁶ to 3 × 10⁶, 3 × 10⁶ to 5 × 10⁶, 5 × 10⁶ to 1 × 10⁷, 1 × 10⁷ to 3 × 10⁷, 3 × 10⁷ to 5 × 10⁷, 5 × 10⁷ to 1 × 10⁸, 1 × 10⁸ to 3 × 10⁸, 3 × 10⁸ to 5 × 10⁸, 5 × 10⁸ to 1 × 10⁹, 1 × 10⁹ to 5 × 10⁹, or 5 × 10⁹ to 1 × 10¹⁰ isolated placental cells (e.g., as part of a pharmaceutical composition comprising placental stem cells). Populations of isolated placental cells useful in the methods of treatment described herein comprise at least 50%, 55%, 60%, 65%, 70%, 75%, 80%, 85%, 90%, 95%, 98%, or 99% viable isolated placental cells, *e.g.,* as determined by, e.g., trypan blue exclusion.

### 4.2 METHODS OF OBTAINING ISOLATED PLACENTAL CELLS

### 4.2.1 Stem Cell Collection Composition

Further provided herein are methods of collecting and isolating placental cells, *e.g.,* the isolated placental cells described in Section 4.1, above. Generally, such cells are obtained from a mammalian placenta using a physiologically-acceptable solution, *e.g.,* a cell collection composition. An exemplary cell collection composition is described in detail in related U.S. Patent Application Publication No. 2007/0190042, entitled "Improved Medium for Collecting Placental Stem Cells and Preserving Organs," the disclosure of which is incorporated herein by reference in its entirety

The cell collection composition can comprise any physiologically-acceptable solution suitable for the collection and/or culture of cells, e.g., the isolated placental cells described herein, for example, a saline solution (e.g., phosphate-buffered saline, Kreb's solution, modified Kreb's solution, Eagle's solution, 0.9% NaCl. etc.), a culture medium (e.g., DMEM, H.DMEM, etc.), and the like.

The cell collection composition can comprise one or more components that tend to preserve isolated placental cells, that is, prevent the isolated placental cells from dying, or delay the death of the isolated placental cells, reduce the number of isolated placental cells in a population of cells that die, or the like, from the time of collection to the time of culturing. Such components can be, e.g., an apoptosis inhibitor (e.g., a caspase inhibitor or JNK inhibitor); a vasodilator (e.g., magnesium sulfate, an antihypertensive drug, atrial natriuretic peptide (ANP), adrenocorticotropin, corticotropin-releasing hormone, sodium nitroprusside, hydralazine, adenosine triphosphate, adenosine, indomethacin or magnesium sulfate, a phosphodiesterase inhibitor, etc.); a necrosis inhibitor (e.g., 2-(1H-Indol-3-yl)-3-pentylamino-maleimide, pyrrolidine dithiocarbamate, or clonazepam); a TNF-α inhibitor; and/or an oxygen-carrying perfluorocarbon (e.g., perfluorooctyl bromide, perfluorodecyl bromide, etc.).

The cell collection composition can comprise one or more tissue-degrading enzymes, e.g., a metalloprotease, a serine protease, a neutral protease, an RNase, or a DNase, or the like. Such enzymes include, but are not limited to, collagenases (e.g., collagenase I, II, III or IV, a collagenase from Clostridium histolyticum, etc.); dispase, thermolysin, elastase, trypsin, LIBERASE, hyaluronidase, and the like.

The cell collection composition can comprise a bacteriocidally or bacteriostatically effective amount of an antibiotic. In certain non-limiting embodiments, the antibiotic is a macrolide (e.g., tobramycin), a cephalosporin (e.g., cephalexin, cephradine, cefuroxime, cefprozil, cefaclor, cefixime or cefadroxil), a clarithromycin, an erythromycin, a penicillin (e.g., penicillin V) or a quinolone (e.g., ofloxacin, ciprofloxacin or norfloxacin), a tetracycline, a streptomycin, etc. In a particular embodiment, the antibiotic is active against Gram(+) and/or Gram(-) bacteria, e.g., Pseudomonas aeruginosa, Staphylococcus aureus, and the like. In one embodiment, the antibiotic is gentamycin, e.g., about 0.005% to about 0.01% (w/v) in culture medium

The cell collection composition can also comprise one or more of the following compounds: adenosine (about 1 mM to about 50 mM); D-glucose (about 20 mM to about 100 mM); magnesium ions (about 1 mM to about 50 mM); a macromolecule of molecular weight greater than 20,000 daltons, in one embodiment, present in an amount sufficient to maintain endothelial integrity and cellular viability (e.g., a synthetic or naturally occurring colloid, a polysaccharide such as dextran or a polyethylene glycol present at about 25 g/l to about 100 g/l, or about 40 g/l to about 60 g/l); an antioxidant (e.g., butylated hydroxyanisole, butylated hydroxytoluene, glutathione, vitamin C or vitamin E present at about 25 µM to about 100 µM); a reducing agent (e.g., N-acetylcysteine present at about 0.1 mM to about 5 mM); an agent that prevents calcium entry into cells (e.g., verapamil present at about 2 µM to about 25 µM); nitroglycerin (e.g., about 0.05 g/L to about 0.2 g/L); an anticoagulant, in one embodiment, present in an amount sufficient to help prevent clotting of residual blood (e.g., heparin or hirudin present at a concentration of about 1000 units/l to about 100,000 units/l); or an amiloride containing compound (e.g., amiloride, ethyl isopropyl amiloride, hexamethylene amiloride, dimethyl amiloride or isobutyl amiloride present at about 1.0 µM to about 5 µM).

### 4.2.2 Collection and Handling of Placenta

Generally, a human placenta is recovered shortly after its expulsion after birth. In a preferred embodiment, the placenta is recovered from a patient after informed consent and after a complete medical history of the patient is taken and is associated with the placenta. Preferably, the medical history continues after delivery. Such a medical history can be used to coordinate subsequent use of the placenta or the isolated placental cells harvested therefrom. For example, isolated human placental cells can be used, in light of the medical history, for personalized medicine for the infant associated with the placenta, or for parents, siblings or other relatives of the infant.

Prior to recovery of isolated placental cells, the umbilical cord blood and placental blood are preferably removed. In certain embodiments, after delivery, the cord blood in the placenta is recovered. The placenta can be subjected to a conventional cord blood recovery process. Typically a needle or cannula is used, with the aid of gravity, to exsanguinate the placenta (see, e.g., Anderson, U.S. Patent No. 5,372,581; Hessel et al., U.S. Patent No. 5,415,665). The needle or cannula is usually placed in the umbilical vein and the placenta can be gently massaged to aid in draining cord blood from the placenta. Such cord blood recovery may be performed commercially, e.g., LifeBank USA, Cedar Knolls, N.J. Preferably, the placenta is gravity drained without further manipulation so as to minimize tissue disruption during cord blood recovery.

Typically, a placenta is transported from the delivery or birthing room to another location, e.g., a laboratory, for recovery of cord blood and collection of stem cells by, e.g., perfusion or tissue dissociation. The placenta is preferably transported in a sterile, thermally insulated transport device (maintaining the temperature of the placenta between 20-28°C), for example, by placing the placenta, with clamped proximal umbilical cord, in a sterile zip-lock plastic bag, which is then placed in an insulated container. In another embodiment, the placenta is transported in a cord blood collection kit substantially as described in pending United States Patent No. 7,147,626, the disclosure of which is incorporated by reference herein. Preferably, the placenta is delivered to the laboratory four to twenty-four hours following delivery. In certain embodiments, the proximal umbilical cord is clamped, preferably within 4-5 cm (centimeter) of the insertion into the placental disc prior to cord blood recovery. In other embodiments, the proximal umbilical cord is clamped after cord blood recovery but prior to further processing of the placenta.

The placenta, prior to cell collection, can be stored under sterile conditions and at either room temperature or at a temperature of 5°C to 25°C. The placenta may be stored for a period of for a period of four to twenty-four hours, up to forty-eight hours, or longer than forty eight hours, prior to perfusing the placenta to remove any residual cord blood. In one embodiment, the placenta is harvested from between about zero hours to about two hours post-expulsion. The placenta is preferably stored in an anticoagulant solution at a temperature of 5°C to 25°C. Suitable anticoagulant solutions are well known in the art. For example, a solution of heparin or warfarin sodium can be used. In a preferred embodiment, the anticoagulant solution comprises a solution of heparin (e.g., 1% w/w in 1:1000 solution). The exsanguinated placenta is preferably stored for no more than 36 hours before placental cells are collected.

The mammalian placenta or a part thereof, once collected and prepared generally as above, can be treated in any art-known manner, *e.g.,* can be perfused or disrupted, *e.g.,* digested with one or more tissue-disrupting enzymes, to obtain isolated placental cells.

### 4.2.3 Physical Disruption and Enzymatic Digestion of Placental Tissue

In one embodiment, stem cells are collected from a mammalian placenta by physical disruption of part of all of the organ. For example, the placenta, or a portion thereof, may be, e.g., crushed, sheared, minced, diced, chopped, macerated or the like. The tissue can then be cultured to obtain a population of isolated placental cells. Typically, the placental tissue is disrupted using, e.g., culture medium, a saline solution, or a stem cell collection.

The placenta can be dissected into components prior to physical disruption and/or enzymatic digestion and stem cell recovery. Isolated placental cells can be obtained from all or a portion of the amniotic membrane, chorion, umbilical cord, placental cotyledons, or any combination thereof, including from a whole placenta. Preferably, isolated placental cells are obtained from placental tissue comprising amnion and chorion. Typically, isolated placental cells can be obtained by disruption of a small block of placental tissue, e.g., a block of placental tissue that is about 1, 2, 3, 4, 5, 6, 7, 8, 9, 10, 20, 30, 40, 50, 60, 70, 80, 90, 100, 200, 300, 400, 500, 600, 700, 800, 900 or about 1000 cubic millimeters in volume. Any method of physical disruption can be used, provided that the method of disruption leaves a plurality, more preferably a majority, and more preferably at least 60%, 70%, 80%, 90%, 95%, 98%, or 99% of the cells in said organ viable, as determined by, e.g., trypan blue exclusion.

The isolated adherent placental cells can generally be collected from a placenta, or portion thereof, at any time within about the first three days post-expulsion, but preferably between about 8 hours and about 18 hours post-expulsion.

In a specific embodiment, the disrupted tissue is cultured in tissue culture medium suitable for the proliferation of isolated placental cells.

In another specific embodiment, isolated placental cells are collected by physical disruption of placental tissue, wherein the physical disruption includes enzymatic digestion, which can be accomplished by use of one or more tissue-digesting enzymes. The placenta, or a portion thereof, may also be physically disrupted and digested with one or more enzymes, and the resulting material then immersed in, or mixed into, a cell collection composition.

A preferred cell collection composition comprises one or more tissue-disruptive enzyme(s). Enzymes that can be used to disrupt placenta tissue include papain, deoxyribonucleases, serine proteases, such as trypsin, chymotrypsin, collagenase, dispase or elastase. Serine proteases may be inhibited by alpha 2 microglobulin in serum and therefore the medium used for digestion is usually serum-free. EDTA and DNase are commonly used in enzyme digestion procedures to increase the efficiency of cell recovery. The digestate is preferably diluted so as to avoid trapping cells within the viscous digest.

Any combination of tissue digestion enzymes can be used. Typical concentrations for digestion using trypsin include, 0.1% to about 2% trypsin, e.g,. about 0.25% trypsin. Proteases can be used in combination, that is, two or more proteases in the same digestion reaction, or can be used sequentially in order to liberate placental cells, e.g., placental stem cells and placental multipotent cells. For example, in one embodiment, a placenta, or part thereof, is digested first with an appropriate amount of collagenase I at about 1 to about 2 mg/ml for, e.g., 30 minutes, followed by digestion with trypsin, at a concentration of about 0.25%, for, e.g., 10 minutes, at 37°C. Serine proteases are preferably used consecutively following use of other enzymes.

In another embodiment, the tissue can further be disrupted by the addition of a chelator, e.g., ethylene glycol bis(2-aminoethyl ether)-N,N,N'N'-tetraacetic acid (EGTA) or ethylenediaminetetraacetic acid (EDTA) to the stem cell collection composition comprising the stem cells, or to a solution in which the tissue is disrupted and/or digested prior to isolation of the stem cells with the stem cell collection composition.

Following digestion, the digestate is washed, for example, three times with culture medium, and the washed cells are seeded into culture flasks. The cells are then isolated by differential adherence, and characterized for, e.g., viability, cell surface markers, differentiation, and the like.

It will be appreciated that where an entire placenta, or portion of a placenta comprising both fetal and maternal cells (for example, where the portion of the placenta comprises the chorion or cotyledons), the placental cells isolated can comprise a mix of placental cells derived from both fetal and maternal sources. Where a portion of the placenta that comprises no, or a negligible number of, maternal cells (for example, amnion), the placental cells isolated therefrom will comprise almost exclusively fetal placental cells (that is, placental cells having the genotype of the fetus).

Placental cells, *e.g.,* the placental cells described in Section 4.1, above, can be isolated from disrupted placental tissue by differential trypsinization (*see* Section 4.2.5, below) followed by culture in one or more new culture containers in fresh proliferation medium, optionally followed by a second differential trypsinization step.

### 4.2.4 Placental Perfusion

Placental cells, *e.g.,* the placental cells described in Section 4.1, above, can also be obtained by perfusion of the mammalian placenta. Methods of perfusing mammalian placenta to obtain placental cells are disclosed, e.g., in Hariri, U.S. Patent Nos. 7,045,148 and 7,255,729, in U.S. Patent Application Publication Nos. 2007/0275362 and 2007/0190042, the disclosures of each of which are incorporated herein by reference in their entireties.

Placental cells can be collected by perfusion, e.g., through the placental vasculature, using, e.g., a cell collection composition as a perfusion solution. In one embodiment, a mammalian placenta is perfused by passage of perfusion solution through either or both of the umbilical artery and umbilical vein. The flow of perfusion solution through the placenta may be accomplished using, e.g., gravity flow into the placenta. Preferably, the perfusion solution is forced through the placenta using a pump, e.g., a peristaltic pump. The umbilical vein can be, e.g., cannulated with a cannula, e.g., a TEFLON^{®} or plastic cannula, that is connected to a sterile connection apparatus, such as sterile tubing. The sterile connection apparatus is connected to a perfusion manifold.

In preparation for perfusion, the placenta is preferably oriented (e.g., suspended) in such a manner that the umbilical artery and umbilical vein are located at the highest point of the placenta. The placenta can be perfused by passage of a perfusion fluid through the placental vasculature and surrounding tissue. The placenta can also be perfused by passage of a perfusion fluid into the umbilical vein and collection from the umbilical arteries, or passage of a perfusion fluid into the umbilical arteries and collection from the umbilical vein.

In one embodiment, for example, the umbilical artery and the umbilical vein are connected simultaneously, e.g., to a pipette that is connected via a flexible connector to a reservoir of the perfusion solution. The perfusion solution is passed into the umbilical vein and artery. The perfusion solution exudes from and/or passes through the walls of the blood vessels into the surrounding tissues of the placenta, and is collected in a suitable open vessel from the surface of the placenta that was attached to the uterus of the mother during gestation. The perfusion solution may also be introduced through the umbilical cord opening and allowed to flow or percolate out of openings in the wall of the placenta which interfaced with the maternal uterine wall. Placental cells that are collected by this method, which can be referred to as a "pan" method, are typically a mixture of fetal and maternal cells.

In another embodiment, the perfusion solution is passed through the umbilical veins and collected from the umbilical artery, or is passed through the umbilical artery and collected from the umbilical veins. Placental cells collected by this method, which can be referred to as a "closed circuit" method, are typically almost exclusively fetal.

It will be appreciated that perfusion using the pan method, that is, whereby perfusate is collected after it has exuded from the maternal side of the placenta, results in a mix of fetal and maternal cells. As a result, the cells collected by this method can comprise a mixed population of placental cells, e.g., placental stem cells or placental multipotent cells, of both fetal and maternal origin. In contrast, perfusion solely through the placental vasculature in the closed circuit method, whereby perfusion fluid is passed through one or two placental vessels and is collected solely through the remaining vessel(s), results in the collection of a population of placental cells almost exclusively of fetal origin.

The closed circuit perfusion method can, in one embodiment, be performed as follows. A post-partum placenta is obtained within about 48 hours after birth. The umbilical cord is clamped and cut above the clamp. The umbilical cord can be discarded, or can processed to recover, e.g., umbilical cord stem cells, and/or to process the umbilical cord membrane for the production of a biomaterial. The amniotic membrane can be retained during perfusion, or can be separated from the chorion, e.g., using blunt dissection with the fingers. If the amniotic membrane is separated from the chorion prior to perfusion, it can be, e.g., discarded, or processed, e.g., to obtain stem cells by enzymatic digestion, or to produce, e.g., an amniotic membrane biomaterial, e.g., the biomaterial described in U.S. Application Publication No. 2004/0048796, the disclosure of which is incorporated by reference herein in its entirety. After cleaning the placenta of all visible blood clots and residual blood, e.g., using sterile gauze, the umbilical cord vessels are exposed, e.g., by partially cutting the umbilical cord membrane to expose a cross-section of the cord. The vessels are identified, and opened, e.g., by advancing a closed alligator clamp through the cut end of each vessel. The apparatus, e.g., plastic tubing connected to a perfusion device or peristaltic pump, is then inserted into each of the placental arteries. The pump can be any pump suitable for the purpose, e.g., a peristaltic pump. Plastic tubing, connected to a sterile collection reservoir, e.g., a blood bag such as a 250 mL collection bag, is then inserted into the placental vein. Alternatively, the tubing connected to the pump is inserted into the placental vein, and tubes to a collection reservoir(s) are inserted into one or both of the placental arteries. The placenta is then perfused with a volume of perfusion solution, e.g., about 750 ml of perfusion solution. Cells in the perfusate are then collected, e.g., by centrifugation. In certain embodiments, the placenta is perfused with perfusion solution, e.g., 100-300 mL perfusion solution, to remove residual blood prior to perfusion to collect placental cells, e.g., placental stem cells and/or placental multipotent cells. In another embodiment, the placenta is not perfused with perfusion solution to remove residual blood prior to perfusion to collect placental cells.

In one embodiment, the proximal umbilical cord is clamped during perfusion, and more preferably, is clamped within 4-5 cm (centimeter) of the cord's insertion into the placental disc.

The first collection of perfusion fluid from a mammalian placenta during the exsanguination process is generally colored with residual red blood cells of the cord blood and/or placental blood. The perfusion fluid becomes more colorless as perfusion proceeds and the residual cord blood cells are washed out of the placenta. Generally from 30 to 100 ml (milliliter) of perfusion fluid is adequate to initially exsanguinate the placenta, but more or less perfusion fluid may be used depending on the observed results.

The volume of perfusion liquid used to isolate placental cells may vary depending upon the number of cells to be collected, the size of the placenta, the number of collections to be made from a single placenta, etc. In various embodiments, the volume of perfusion liquid may be from 50 mL to 5000 mL, 50 mL to 4000 mL, 50 mL to 3000 mL, 100 mL to 2000 mL, 250 mL to 2000 mL, 500 mL to 2000 mL, or 750 mL to 2000 mL. Typically, the placenta is perfused with 700-800 mL of perfusion liquid following exsanguination.

The placenta can be perfused a plurality of times over the course of several hours or several days. Where the placenta is to be perfused a plurality of times, it may be maintained or cultured under aseptic conditions in a container or other suitable vessel, and perfused with the cell collection composition, or a standard perfusion solution (e.g., a normal saline solution such as phosphate buffered saline ("PBS")) with or without an anticoagulant (e.g., heparin, warfarin sodium, coumarin, bishydroxycoumarin), and/or with or without an antimicrobial agent (e.g., β-mercaptoethanol (0.1 mM); antibiotics such as streptomycin (e.g., at 40-100 µg/ml), penicillin (e.g., at 40U/ml), amphotericin B (e.g., at 0.5 µg/ml). In one embodiment, an isolated placenta is maintained or cultured for a period of time without collecting the perfusate, such that the placenta is maintained or cultured for 1, 2, 3, 4, 5, 6, 7, 8, 9, 10, 11, 12, 13, 14, 15, 16, 17, 18, 19, 20, 21, 22, 23, or 24 hours, or 2 or 3 or more days before perfusion and collection of perfusate. The perfused placenta can be maintained for one or more additional time(s), e.g., 1, 2, 3, 4, 5, 6, 7, 8, 9, 10, 11, 12, 13, 14, 15, 16, 17, 18, 19, 20, 21, 22, 23, 24 or more hours, and perfused a second time with, e.g., 700-800 mL perfusion fluid. The placenta can be perfused 1, 2, 3, 4, 5 or more times, for example, once every 1, 2, 3, 4, 5 or 6 hours. In a preferred embodiment, perfusion of the placenta and collection of perfusion solution, e.g., cell collection composition, is repeated until the number of recovered nucleated cells falls below 100 cells/ml. The perfusates at different time points can be further processed individually to recover time-dependent populations of cells, e.g., stem cells. Perfusates from different time points can also be pooled. In a preferred embodiment, placental cells are collected at a time or times between about 8 hours and about 18 hours post-expulsion.

Perfusion preferably results in the collection of significantly more placental cells than the number obtainable from a mammalian placenta not perfused with said solution, and not otherwise treated to obtain placental cells (e.g., by tissue disruption, e.g., enzymatic digestion). In this context, "significantly more" means at least 10% more. Perfusion yields significantly more placental cells than, e.g., the number of placental cells isolatable from culture medium in which a placenta, or portion thereof, has been cultured.

Placental cells can be isolated from placenta by perfusion with a solution comprising one or more proteases or other tissue-disruptive enzymes. In a specific embodiment, a placenta or portion thereof (e.g., amniotic membrane, amnion and chorion, placental lobule or cotyledon, umbilical cord, or combination of any of the foregoing) is brought to 25-37°C, and is incubated with one or more tissue-disruptive enzymes in 200 mL of a culture medium for 30 minutes. Cells from the perfusate are collected, brought to 4°C, and washed with a cold inhibitor mix comprising 5 mM EDTA, 2 mM dithiothreitol and 2 mM beta-mercaptoethanol. The placental cells are washed after several minutes with a cold *(e.g.,* 4°C) stem cell collection composition.

### 4.2.5 Isolation, Sorting, and Characterization of Placental Cells

The isolated placental cells, *e.g.,* the cells described in Section 4.1, above, whether obtained by perfusion or physical disruption, e.g., by enzymatic digestion, can initially be purified from (*i.e.,* be isolated from) other cells by Ficoll gradient centrifugation. Such centrifugation can follow any standard protocol for centrifugation speed, etc. In one embodiment, for example, cells collected from the placenta are recovered from perfusate by centrifugation at 5000 × g for 15 minutes at room temperature, which separates cells from, e.g., contaminating debris and platelets. In another embodiment, placental perfusate is concentrated to about 200 ml, gently layered over Ficoll, and centrifuged at about 1100 × g for 20 minutes at 22°C, and the low-density interface layer of cells is collected for further processing.

Cell pellets can be resuspended in fresh stem cell collection composition, or a medium suitable for cell maintenance, e.g., stem cell maintenance, for example, IMDM serum-free medium containing 2U/ml heparin and 2 mM EDTA (GibcoBRL, NY). The total mononuclear cell fraction can be isolated, e.g., using Lymphoprep (Nycomed Pharma, Oslo, Norway) according to the manufacturer's recommended procedure.

Placental cells obtained by perfusion or digestion can, for example, be further, or initially, isolated by differential trypsinization using, e.g., a solution of 0.05% trypsin with 0.2% EDTA (Sigma, St. Louis MO). Differential trypsinization is possible because the isolated placental cells, which are tissue culture plastic-adherent, typically detach from the plastic surfaces within about five minutes whereas other adherent populations typically require more than 20-30 minutes incubation. The detached placental cells can be harvested following trypsinization and trypsin neutralization, using, e.g., Trypsin Neutralizing Solution (TNS, Cambrex). In one embodiment of isolation of adherent cells, aliquots of, for example, about 5-10 × 10⁶ cells are placed in each of several T-75 flasks, preferably fibronectin-coated T75 flasks. In such an embodiment, the cells can be cultured with commercially available Mesenchymal Stem Cell Growth Medium (MSCGM) (Cambrex), and placed in a tissue culture incubator (37°C, 5% CO2). After 10 to 15 days, non-adherent cells are removed from the flasks by washing with PBS. The PBS is then replaced by MSCGM. Flasks are preferably examined daily for the presence of various adherent cell types and in particular, for identification and expansion of clusters of fibroblastoid cells.

The number and type of cells collected from a mammalian placenta can be monitored, for example, by measuring changes in morphology and cell surface markers using standard cell detection techniques such as flow cytometry, cell sorting, immunocytochemistry (e.g., staining with tissue specific or cell-marker specific antibodies) fluorescence activated cell sorting (FACS), magnetic activated cell sorting (MACS), by examination of the morphology of cells using light or confocal microscopy, and/or by measuring changes in gene expression using techniques well known in the art, such as PCR and gene expression profiling. These techniques can be used, too, to identify cells that are positive for one or more particular markers. For example, using antibodies to CD34, one can determine, using the techniques above, whether a cell comprises a detectable amount of CD34; if so, the cell is CD34+. Likewise, if a cell produces enough OCT-4 RNA to be detectable by RT-PCR, or significantly more OCT-4 RNA than an adult cell, the cell is OCT-4+. Antibodies to cell surface markers (e.g., CD markers such as CD34) and the sequence of stem cell-specific genes, such as OCT-4, are well-known in the art.

Placental cells, particularly cells that have been isolated by Ficoll separation, differential adherence, or a combination of both, may be sorted using a fluorescence activated cell sorter (FACS). Fluorescence activated cell sorting (FACS) is a well-known method for separating particles, including cells, based on the fluorescent properties of the particles (Kamarch, 1987, Methods Enzymol, 151:150-165). Laser excitation of fluorescent moieties in the individual particles results in a small electrical charge allowing electromagnetic separation of positive and negative particles from a mixture. In one embodiment, cell surface marker-specific antibodies or ligands are labeled with distinct fluorescent labels. Cells are processed through the cell sorter, allowing separation of cells based on their ability to bind to the antibodies used. FACS sorted particles may be directly deposited into individual wells of 96-well or 384-well plates to facilitate separation and cloning.

In one sorting scheme, cells from placenta, e.g., PDACs are sorted on the basis of expression of one or more of the markers CD34, CD38, CD44, CD45, CD73, CD105, OCT-4 and/or HLA-G. This can be accomplished in connection with procedures to select such cells on the basis of their adherence properties in culture. For example, tissue culture plastic adherence selection can be accomplished before or after sorting on the basis of marker expression. In one embodiment, for example, cells are sorted first on the basis of their expression of CD34; CD34-cells are retained, and CD34- cells that are additionally CD200+ and HLA-G― are separated from all other CD34- cells. In another embodiment, cells from placenta are sorted based on their expression of markers CD200 and/or HLA-G; for example, cells displaying CD200 and lacking HLA-G are isolated for further use. Cells that express, e.g., CD200 and/or lack, e.g., HLA-G can, in a specific embodiment, be further sorted based on their expression of CD73 and/or CD105, or epitopes recognized by antibodies SH2, SH3 or SH4, or lack of expression of CD34, CD38 or CD45. For example, in another embodiment, placental cells are sorted by expression, or lack thereof, of CD200, HLA-G, CD73, CD105, CD34, CD38 and CD45, and placental cells that are CD200+, HLA-G-, CD73+, CD105+, CD34-, CD38- and CD45- are isolated from other placental cells for further use.

In specific embodiments of any of the above embodiments of sorted placental cells, at least 50%, 60%, 70%, 80%, 90% or 95% of the cells in a cell population remaining after sorting are said isolated placental cells. Placental cells can be sorted by one or more of any of the markers described in Section 4.1, above.

In a specific embodiment, for example, placental cells that are (1) adherent to tissue culture plastic, and (2) CD10+, CD34- and CD105+ are sorted from (i.e., isolated from) other placental cells. In another specific embodiment, placental cells that are (1) adherent to tissue culture plastic, and (2) CD10+, CD34-, CD105+ and CD200+ are sorted from (i.e., isolated from) other placental cells. In another specific embodiment, placental cells that are (1) adherent to tissue culture plastic, and (2) CD10+, CD34-, CD45-, CD90+, CD105+ and CD200+ are sorted from (i.e., isolated from) other placental cells.

With respect to nucleotide sequence-based detection of placental cells, sequences for the markers listed herein are readily available in publicly-available databases such as GenBank or EMBL.

With respect to antibody-mediated detection and sorting of placental cells, e.g., placental stem cells or placental multipotent cells, any antibody, specific for a particular marker, can be used, in combination with any fluorophore or other label suitable for the detection and sorting of cells (e.g., fluorescence-activated cell sorting). Antibody/fluorophore combinations to specific markers include, but are not limited to, fluorescein isothiocyanate (FITC) conjugated monoclonal antibodies against HLA-G (available from Serotec, Raleigh, North Carolina), CD10 (available from BD Immunocytometry Systems, San Jose, California), CD44 (available from BD Biosciences Pharmingen, San Jose, California), and CD105 (available from R&D Systems Inc., Minneapolis, Minnesota); phycoerythrin (PE) conjugated monoclonal antibodies against CD44, CD200, CD117, and CD13 (BD Biosciences Pharmingen); phycoerythrin-Cy7 (PE Cy7) conjugated monoclonal antibodies against CD33 and CD10 (BD Biosciences Pharmingen); allophycocyanin (APC) conjugated streptavidin and monoclonal antibodies against CD38 (BD Biosciences Pharmingen); and Biotinylated CD90 (BD Biosciences Pharmingen). Other antibodies that can be used include, but are not limited to, CD133-APC (Miltenyi), KDR-Biotin (CD309, Abcam), CytokeratinK-Fitc (Sigma or Dako), HLA ABC-Fitc (BD), HLA DR,DQ,DP-PE (BD), β-2-microglobulin-PE (BD), CD80-PE (BD) and CD86-APC (BD). Other antibody/label combinations that can be used include, but are not limited to, CD45-PerCP (peridin chlorophyll protein); CD44-PE; CD19-PE; CD10-F (fluorescein); HLA-G-F and 7-amino-actinomycin-D (7-AAD); HLA-ABC-F; and the like. This list is not exhaustive, and other antibodies from other suppliers are also commercially available.

The isolated placental cells provided herein can be assayed for CD117 or CD133 using, for example, phycoerythrin-Cy5 (PE Cy5) conjugated streptavidin and biotin conjugated monoclonal antibodies against CD117 or CD133; however, using this system, the cells can appear to be positive for CD117 or CD133, respectively, because of a relatively high background.

The isolated placental cells can be labeled with an antibody to a single marker and detected and/sorted. Placental cells can also be simultaneously labeled with multiple antibodies to different markers.

In another embodiment, magnetic beads can be used to separate cells. The cells may be sorted using a magnetic activated cell sorting (MACS) technique, a method for separating particles based on their ability to bind magnetic beads (0.5-100 µm diameter). A variety of useful modifications can be performed on the magnetic microspheres, including covalent addition of antibody that specifically recognizes a particular cell surface molecule or hapten. The beads are then mixed with the cells to allow binding. Cells are then passed through a magnetic field to separate out cells having the specific cell surface marker. In one embodiment, these cells can then isolated and re-mixed with magnetic beads coupled to an antibody against additional cell surface markers. The cells are again passed through a magnetic field, isolating cells that bound both the antibodies. Such cells can then be diluted into separate dishes, such as microtiter dishes for clonal isolation.

Isolated placental cells can also be characterized and/or sorted based on cell morphology and growth characteristics. For example, isolated placental cells can be characterized as having, and/or selected on the basis of, e.g., a fibroblastoid appearance in culture. The isolated placental cells can also be characterized as having, and/or be selected, on the basis of their ability to form embryoid-like bodies. In one embodiment, for example, placental cells that are fibroblastoid in shape, express CD73 and CD105, and produce one or more embryoid-like bodies in culture are isolated from other placental cells. In another embodiment, OCT-4+ placental cells that produce one or more embryoid-like bodies in culture are isolated from other placental cells.

In another embodiment, isolated placental cells can be identified and characterized by a colony forming unit assay. Colony forming unit assays are commonly known in the art, such as MesenCult^{™} medium (Stem Cell Technologies, Inc., Vancouver British Columbia).

The isolated placental cells can be assessed for viability, proliferation potential, and longevity using standard techniques known in the art, such as trypan blue exclusion assay, fluorescein diacetate uptake assay, propidium iodide uptake assay (to assess viability); and thymidine uptake assay, MTT (3-(4,5-Dimethylthiazol-2-yl)-2,5-diphenyltetrazolium bromide) cell proliferation assay (to assess proliferation). Longevity may be determined by methods well known in the art, such as by determining the maximum number of population doubling in an extended culture.

Isolated placental cells, e.g., the isolated placental cells described in Section 4.1, above, can also be separated from other placental cells using other techniques known in the art, e.g., selective growth of desired cells (positive selection), selective destruction of unwanted cells (negative selection); separation based upon differential cell agglutinability in the mixed population as, for example, with soybean agglutinin; freeze-thaw procedures; filtration; conventional and zonal centrifugation; centrifugal elutriation (counter-streaming centrifugation); unit gravity separation; countercurrent distribution; electrophoresis; and the like.

### 4.2.6 Populations of Isolated Placental Cells

Also provided herein are populations of isolated placental cells, *e.g.,* the isolated placental cells described in Section 4.1, above, useful in the methods and compositions described herein. Populations of isolated placental cells can be isolated directly from one or more placentas; that is, the cell population can be a population of placental cells comprising the isolated placental cells, wherein the isolated placental cells are obtained from, or contained within, perfusate, or obtained from, or contained within, disrupted placental tissue, e.g., placental tissue digestate (that is, the collection of cells obtained by enzymatic digestion of a placenta or part thereof). The isolated placental cells described herein can also be cultured and expanded to produce populations of the isolated placental cells. Populations of placental cells comprising the isolated placental cells can also be cultured and expanded to produce placental cell populations.

Placental cell populations useful in the methods of treatment provided herein comprise the isolated placental cells, for example, the isolated placental cells as described in Section 4.1 herein. In various embodiments, at least 10%, 20%, 30%, 40%, 50%, 60%, 70%, 80%, 90%, 95%, or 99% of the cells in a placental cell population are the isolated placental cells. That is, a population of the isolated placental cells can comprise, e.g., as much as 1%, 5%, 10%, 20%, 30%, 40%, 50%, 60%, 70%, 80%, 90% cells that are not the isolated placental cells.

Isolated placental cell populations useful in the methods and compositions described herein can be produced by, e.g., selecting isolated placental cells, whether derived from enzymatic digestion or perfusion, that express particular markers and/or particular culture or morphological characteristics. In one embodiment, for example, provided herein is a method of producing a cell population by selecting placental cells that (a) adhere to a substrate, and (b) express CD200 and lack expression of HLA-G; and isolating said cells from other cells to form a cell population. In another embodiment, a cell population is produced by selecting placental cells that express CD200 and lack expression of HLA-G, and isolating said cells from other cells to form a cell population. In another embodiment, a cell population is produced by selecting placental cells that (a) adhere to a substrate, and (b) express CD73, CD105, and CD200; and isolating said cells from other cells to form a cell population. In another embodiment, a cell population is produced by identifying placental cells that express CD73, CD105, and CD200, and isolating said cells from other cells to form a cell population. In another embodiment, a cell population is produced by selecting placental cells that (a) adhere to a substrate and (b) express CD200 and OCT-4; and isolating said cells from other cells to form a cell population. In another embodiment, a cell population is produced by selecting placental cells that express CD200 and OCT-4, and isolating said cells from other cells to form a cell population. In another embodiment, a cell population is produced by selecting placental cells that (a) adhere to a substrate, (b) express CD73 and CD105, and (c) facilitate the formation of one or more embryoid-like bodies in a population of placental cells comprising said stem cell when said population is cultured under conditions that allow for the formation of an embryoid-like body; and isolating said cells from other cells to form a cell population. In another embodiment, a cell population is produced by selecting placental cells that express CD73 and CD105, and facilitate the formation of one or more embryoid-like bodies in a population of placental cells comprising said stem cell when said population is cultured under conditions that allow for the formation of an embryoid-like body, and isolating said cells from other cells to form a cell population. In another embodiment, a cell population is produced by selecting placental cells that (a) adhere to a substrate, and (b) express CD73 and CD105, and lack expression of HLA-G; and isolating said cells from other cells to form a cell population. In another embodiment, a cell population is produced by selecting placental cells that express CD73 and CD105 and lack expression of HLA-G, and isolating said cells from other cells to form a cell population. In another embodiment, the method of producing a cell population comprises selecting placental cells that (a) adhere to a substrate, (b) express OCT-4, and (c) facilitate the formation of one or more embryoid-like bodies in a population of placental cells comprising said stem cell when said population is cultured under conditions that allow for the formation of an embryoid-like body; and isolating said cells from other cells to form a cell population. In another embodiment, a cell population is produced by selecting placental cells that express OCT-4, and facilitate the formation of one or more embryoid-like bodies in a population of placental cells comprising said stem cell when said population is cultured under conditions that allow for the formation of an embryoid-like body, and isolating said cells from other cells to form a cell population.

In another embodiment, a cell population is produced by selecting placental cells that (a) adhere to a substrate, and (b) express CD10 and CD105, and do not express CD34; and isolating said cells from other cells to form a cell population. In another embodiment, a cell population is produced by selecting placental cells that express CD10 and CD105, and do not express CD34, and isolating said cells from other cells to form a cell population. In another embodiment, a cell population is produced by selecting placental cells that (a) adhere to a substrate, and (b) express CD10, CD105, and CD200, and do not express CD34; and isolating said cells from other cells to form a cell population. In another embodiment, a cell population is produced by selecting placental cells that express CD10, CD105, and CD200, and do not express CD34, and isolating said cells from other cells to form a cell population. In another specific embodiment, a cell population is produced by selecting placental cells that (a) adhere to a substrate, and (b) express CD10, CD90, CD105 and CD200, and do not express CD34 and CD45; and isolating said cells from other cells to form a cell population. In another specific embodiment, a cell population is produced by selecting placental cells that express CD10, CD90, CD105 and CD200, and do not express CD34 and CD45, and isolating said cells from other cells to form a cell population.

Selection of cell populations comprising placental cells having any of the marker combinations described in Section 4.1, above, can be isolated or obtained in similar fashion.

In any of the above embodiments, selection of the isolated cell populations can additionally comprise selecting placental cells that express ABC-p (a placenta-specific ABC transporter protein; see, e.g., Allikmets et al., Cancer Res. 58(23):5337-9 (1998)). The method can also comprise selecting cells exhibiting at least one characteristic specific to, e.g., a mesenchymal stem cell, for example, expression of CD44, expression of CD90, or expression of a combination of the foregoing.

In the above embodiments, the substrate can be any surface on which culture and/or selection of cells, e.g., isolated placental cells, can be accomplished. Typically, the substrate is plastic, e.g., tissue culture dish or multiwell plate plastic. Tissue culture plastic can be coated with a biomolecule, e.g., laminin or fibronectin.

Cells, e.g., isolated placental cells, can be selected for a placental cell population by any means known in the art of cell selection. For example, cells can be selected using an antibody or antibodies to one or more cell surface markers, for example, in flow cytometry or FACS. Selection can be accomplished using antibodies in conjunction with magnetic beads. Antibodies that are specific for certain stem cell-related markers are known in the art. For example, antibodies to OCT-4 (Abcam, Cambridge, MA), CD200 (Abcam), HLA-G (Abcam), CD73 (BD Biosciences Pharmingen, San Diego, CA), CD105 (Abcam; BioDesign International, Saco, ME), etc. Antibodies to other markers are also available commercially, e.g., CD34, CD38 and CD45 are available from, e.g., StemCell Technologies or BioDesign International.

The isolated placental cell populations can comprise placental cells that are not stem cells, or cells that are not placental cells.

The isolated cell populations comprising placental derived adherent cells described herein can comprise a second cell type, e.g., placental cells that are not placental derived adherent cells, or, e.g., cells that are not placental cells. For example, an isolated population of placental derived adherent cells can comprise, e.g., can be combined with, a population of a second type of cells, wherein said second type of cell are, e.g., embryonic stem cells, blood cells (e.g., placental blood, placental blood cells, umbilical cord blood, umbilical cord blood cells, peripheral blood, peripheral blood cells, nucleated cells from placental blood, umbilical cord blood, or peripheral blood, and the like), stem cells isolated from blood (e.g., stem cells isolated from placental blood, umbilical cord blood or peripheral blood), nucleated cells from placental perfusate, e.g., total nucleated cells from placental perfusate; umbilical cord stem cells, populations of blood-derived nucleated cells, bone marrow-derived mesenchymal stromal cells, bone marrow-derived mesenchymal stem cells, bone marrow-derived hematopoietic stem cells, crude bone marrow, adult (somatic) stem cells, populations of stem cells contained within tissue, cultured cells, e.g., cultured stem cells, populations of fully-differentiated cells (e.g., chondrocytes, fibroblasts, amniotic cells, osteoblasts, muscle cells, cardiac cells, etc.), pericytes, and the like. In a specific embodiment, a population of cells comprising placental derived adherent cells comprises placental stem cells or stem cells from umbilical cord. In certain embodiments in which the second type of cell is blood or blood cells, erythrocytes have been removed from the population of cells.

In a specific embodiment, the second type of cell is a hematopoietic stem cell. Such hematopoietic stem cells can be, for example, contained within unprocessed placental, umbilical cord blood or peripheral blood; in total nucleated cells from placental blood, umbilical cord blood or peripheral blood; in an isolated population of CD34+ cells from placental blood, umbilical cord blood or peripheral blood; in unprocessed bone marrow; in total nucleated cells from bone marrow; in an isolated population of CD34+ cells from bone marrow, or the like.

In another embodiment, an isolated population of placental derived adherent cells is combined with a plurality of adult or progenitor cells from the vascular system. In various embodiments, the cells are endothelial cells, endothelial progenitor cells, myocytes, cardiomyocytes, pericytes, angioblasts, myoblasts or cardiomyoblasts.

In a another embodiment, the second cell type is a non-embryonic cell type manipulated in culture in order to express markers of pluripotency and functions associated with embryonic stem cells

In specific embodiments of the above isolated populations of placental derived adherent cells, either or both of the placental derived adherent cells and cells of a second type are autologous, or are allogeneic, to an intended recipient of the cells.

In another specific embodiment, the composition comprises placental derived adherent cells, and embryonic stem cells. In another specific embodiment, the composition comprises placental derived adherent cells and mesenchymal stromal or stem cells, e.g., bone marrow-derived mesenchymal stromal or stem cells. In another specific embodiment, the composition comprises bone marrow-derived hematopoietic stem cells. In another specific embodiment, the composition comprises placental derived adherent cells and hematopoietic progenitor cells, e.g., hematopoietic progenitor cells from bone marrow, fetal blood, umbilical cord blood, placental blood, and/or peripheral blood. In another specific embodiment, the composition comprises placental derived adherent cells and somatic stem cells. In a more specific embodiment, said somatic stem cell is a neural stem cell, a hepatic stem cell, a pancreatic stem cell, an endothelial stem cell, a cardiac stem cell, or a muscle stem cell.

In other specific embodiments, the second type of cells comprise about, at least, or no more than, 10%, 15%, 20%, 25%, 30%, 35%, 40%, 45%, or 50% of cells in said population. In other specific embodiments, the PDAC in said composition comprise at least 50%, 55%, 60%, 65%, 70%, 75%, 80%, 85% or 90% of cells in said composition. In other specific embodiments, the placental derived adherent cells comprise about, at least, or no more than, 10%, 15%, 20%, 25%, 30%, 35%, 40%, or 45% of cells in said population.

Cells in an isolated population of placental derived adherent cells can be combined with a plurality of cells of another type, e.g., with a population of stem cells, in a ratio of about 100,000,000:1, 50,000,000:1, 20,000,000:1, 10,000,000:1, 5,000,000:1, 2,000,000:1, 1,000,000:1, 500,000:1, 200,000:1, 100,000:1, 50,000:1, 20,000:1, 10,000:1, 5,000:1, 2,000:1, 1,000:1, 500:1, 200:1, 100:1, 50:1, 20:1, 10:1, 5:1, 2:1, 1:1; 1:2; 1:5; 1:10; 1:100; 1:200; 1:500; 1:1,000; 1:2,000; 1:5,000; 1:10,000; 1:20,000; 1:50,000; 1:100,000; 1:500,000; 1:1,000,000; 1:2,000,000; 1:5,000,000; 1:10,000,000; 1:20,000,000; 1:50,000,000; or about 1:100,000,000, comparing numbers of total nucleated cells in each population. Cells in an isolated population of placental derived adherent cells can be combined with a plurality of cells of a plurality of cell types, as well.

In other embodiments, a population of the placental cells described herein, e.g., the PDACs described above, are combined with osteogenic placental adherent cells (OPACs), *e.g.,* the OPACs described in Patent Application No. 12/546,556, filed August 24, 2009, entitled "Methods and Compositions for Treatment of Bone Defects With Placental Stem Cells," or combined with amnion-derived angiogenic cells (AMDACs), e.g., the AMDACs described in U.S. Patent Application No. 12/622,352, entitled "Amnion Derived Angiogenic Cells", the disclosure of which is hereby incorporated by reference in its entirety.

### 4.3 COMPOSITIONS COMPRISING ISOLATED PLACENTAL CELLS

The placental cells described herein, *e.g.,* in Section 4.1, can be combined with any physiologically-acceptable or medically-acceptable compound, composition or device for use in the methods and compositions described herein. Compositions useful in the methods of treatment provided herein can comprise any one or more of the placental cells described herein. In certain embodiments, the composition is a pharmaceutically-acceptable composition, e.g., a composition comprising placental cells in a pharmaceutically-acceptable carrier.

In certain embodiments, a composition comprising the isolated placental cells additionally comprises a matrix, e.g., a decellularized matrix or a synthetic matrix. In another specific embodiment, said matrix is a three-dimensional scaffold. In another specific embodiment, said matrix comprises collagen, gelatin, laminin, fibronectin, pectin, ornithine, or vitronectin. In another ore specific embodiment, the matrix is an amniotic membrane or an amniotic membrane-derived biomaterial. In another specific embodiment, said matrix comprises an extracellular membrane protein. In another specific embodiment, said matrix comprises a synthetic compound. In another specific embodiment, said matrix comprises a bioactive compound. In another specific embodiment, said bioactive compound is a growth factor, cytokine, antibody, or organic molecule of less than 5,000 daltons.

In another embodiment, a composition useful in the methods of treatment provided herein comprises medium conditioned by any of the foregoing placental cells, or any of the foregoing placental cell populations.

### 4.3.1 Cryopreserved Isolated Placental Cells

The isolated placental cell populations useful in the methods and compositions described herein can be preserved, for example, cryopreserved for later use. Methods for cryopreservation of cells, such as stem cells, are well known in the art. Isolated placental cell populations can be prepared in a form that is easily administrable to an individual, e.g., an isolated placental cell population that is contained within a container that is suitable for medical use. Such a container can be, for example, a syringe, sterile plastic bag, flask, jar, or other container from which the isolated placental cell population can be easily dispensed. For example, the container can be a blood bag or other plastic, medically-acceptable bag suitable for the intravenous administration of a liquid to a recipient. The container, in certain embodiments, is one that allows for cryopreservation of the combined cell population.

The cryopreserved isolated placental cell population can comprise isolated placental cell derived from a single donor, or from multiple donors. The isolated placental cell population can be completely HLA-matched to an intended recipient, or partially or completely HLA-mismatched.

Thus, in one embodiment, isolated placental cells can be used in the methods and described herein in the form of a composition comprising a tissue culture plastic-adherent placental cell population in a container. In a specific embodiment, the isolated placental cells are cryopreserved. In another specific embodiment, the container is a bag, flask, or jar. In another specific embodiment, said bag is a sterile plastic bag. In another specific embodiment, said bag is suitable for, allows or facilitates intravenous administration of said isolated placental cell population, e.g., by intravenous infusion. The bag can comprise multiple lumens or compartments that are interconnected to allow mixing of the isolated placental cells and one or more other solutions, e.g., a drug, prior to, or during, administration. In another specific embodiment, the composition comprises one or more compounds that facilitate cryopreservation of the combined cell population. In another specific embodiment, said isolated placental cell population is contained within a physiologically-acceptable aqueous solution. In another specific embodiment, said physiologically-acceptable aqueous solution is a 0.9% NaCl solution. In another specific embodiment, said isolated placental cell population comprises placental cells that are HLA-matched to a recipient of said cell population. In another specific embodiment, said combined cell population comprises placental cells that are at least partially HLA-mismatched to a recipient of said cell population. In another specific embodiment, said isolated placental cells are derived from a plurality of donors.

In certain embodiments, the isolated placental cells in the container are isolated CD10+, CD34-, CD105+ placental cells, wherein said cells have been cryopreserved, and are contained within a container. In a specific embodiment, said CD10+, CD34-, CD105+ placental cells are also CD200+. In another specific embodiment, said CD10+, CD34-, CD105+, CD200+ placental cells are also CD45- or CD90+. In another specific embodiment, said CD10+, CD34-, CD105+, CD200+ placental cells are also CD45- and CD90+. In another specific embodiment, the CD34-, CD10+, CD105+ placental cells are additionally one or more of CD13+, CD29+, CD33+, CD38-, CD44+, CD45-, CD54+, CD62E-, CD62L-, CD62P-, SH3+ (CD73+), SH4+ (CD73+), CD80-, CD86-, CD90+, SH2+ (CD105+), CD106/VCAM+, CD117―, CD144/VE-cadherindim, CD184/CXCR4-, CD200+, CD133-, OCT-4+, SSEA3-, SSEA4-, ABC-p+, KDR- (VEGFR2-), HLA-A,B,C+, HLA-DP,DQ,DR―, HLA-G-, or Programmed Death-1 Ligand (PDL1)+, or any combination thereof. In another specific embodiment, the CD34-, CD10+, CD105+ placental cells are additionally CD13+, CD29+, CD33+, CD38-, CD44+, CD45-, CD54/ICAM+, CD62E-, CD62L-, CD62P-, SH3+ (CD73+), SH4+ (CD73+), CD80―, CD86-, CD90+, SH2+ (CD105+), CD106/VCAM+, CD117―, CD144/VE-cadherindim, CD184/CXCR4―, CD200+, CD133-, OCT-4+, SSEA3-, SSEA4-, ABC-p+, KDR- (VEGFR2― ), HLA-A,B,C+, HLA-DP,DQ,DR―, HLA-G-, and Programmed Death-1 Ligand (PDL1)+.

In certain other embodiments, the above-referenced isolated placental cells are isolated CD200+, HLA-G― placental cells, wherein said cells have been cryopreserved, and are contained within a container. In another embodiment, the isolated placental cells are CD73+, CD105+, CD200+ cells that have been cryopreserved, and are contained within a container. In another embodiment, the isolated placental cells are CD200+, OCT-4+ stem cells that have been cryopreserved, and are contained within a container. In another embodiment, the isolated placental cells are CD73+, CD105+ cells that have been cryopreserved, and are contained within a container, and wherein said isolated placental cells facilitate the formation of one or more embryoid-like bodies when cultured with a population of placental cells under conditions that allow for the formation of embryoid-like bodies. In another embodiment, the isolated placental cells are CD73+, CD105+, HLA-G― cells that have been cryopreserved, and are contained within a container. In another embodiment, the isolated placental cells are OCT-4+ placental cells that have been cryopreserved, and are contained within a container, and wherein said cells facilitate the formation of one or more embryoid-like bodies when cultured with a population of placental cells under conditions that allow for the formation of embryoid-like bodies.

In another specific embodiment, the above-referenced isolated placental cells are placental stem cells or placental multipotent cells that are CD34-, CD10+ and CD105+ as detected by flow cytometry (e.g., PDACs). In another specific embodiment, the isolated CD34-, CD10+, CD105+ placental cells have the potential to differentiate into cells of a neural phenotype, cells of an osteogenic phenotype, or cells of a chondrogenic phenotype. In another specific embodiment, the isolated CD34-, CD10+, CD105+ placental cells are additionally CD200+. In another specific embodiment, the isolated CD34-, CD10+, CD105+ placental cells are additionally CD90+ or CD45-, as detected by flow cytometry. In another specific embodiment, the isolated CD34-, CD10+, CD105+ placental cells are additionally CD90+ or CD45-, as detected by flow cytometry. In another specific embodiment, the CD34-, CD10+, CD105+, CD200+ placental cells are additionally CD90+ or CD45-, as detected by flow cytometry. In another specific embodiment, the CD34-, CD10+, CD105+, CD200+ cells are additionally CD90+ and CD45-, as detected by flow cytometry. In another specific embodiment, the CD34-, CD10+, CD105+, CD200+, CD90+, CD45- cells are additionally CD80- and CD86-, as detected by flow cytometry. In another specific embodiment, the CD34-, CD10+, CD105+ cells are additionally one or more of CD29+, CD38-, CD44+, CD54+, CD80―, CD86-, SH3+ or SH4+. In another specific embodiment, the cells are additionally CD44+. In a specific embodiment of any of the isolated CD34-, CD10+, CD105+ placental cells above, the cells are additionally one or more of CD117―, CD133-, KDR- (VEGFR2-), HLA-A,B,C+, HLA-DP,DQ,DR-, and/or PDL1+.

In a specific embodiment of any of the foregoing cryopreserved isolated placental cells, said container is a bag. In various specific embodiments, said container comprises about, at least, or at most 1 × 10⁶ said isolated placental cells, 5 × 10⁶ said isolated placental cells, 1 × 10⁷ said isolated placental cells, 5 × 10⁷ said isolated placental cells, 1 × 10⁸ said isolated placental cells, 5 × 10⁸ said isolated placental cells, 1 × 10⁹ said isolated placental cells, 5 × 10⁹ said isolated placental cells, 1 × 10¹⁰ said isolated placental cells, or 1 × 10¹⁰ said isolated placental cells. In other specific embodiments of any of the foregoing cryopreserved populations, said isolated placental cells have been passaged about, at least, or no more than 5 times, no more than 10 times, no more than 15 times, or no more than 20 times. In another specific embodiment of any of the foregoing cryopreserved isolated placental cells, said isolated placental cells have been expanded within said container.

In certain embodiments, a single unit dose of placental derived adherent cells can comprise, in various embodiments, about, at least, or no more than 1 × 10³, 3 × 10³, 5 × 10³, 1 × 10⁴, 3 × 10⁴, 5 × 10⁴, 1 × 10⁵ 3 × 10⁵, 5 × 10⁵, 1 × 10⁶, 3 × 10⁶, 5 × 10⁶, 1 × 10⁷, 3 × 10⁷, 5 × 10⁷, 1 × 10⁸, 3 × 10⁸, 5 × 10⁸, 1 × 10⁹, 5 × 10⁹, or 1 × 10¹⁰ placental cells. In certain embodiments, a single unit dose of placental derived adherent cells can comprise between 1 × 10³ to 3 × 10³, 3 × 10³ to 5 × 10³, 5 × 10³ to 1 × 10⁴, 1 × 10⁴ to 3 × 10⁴, 3 × 10⁴ to 5 × 10⁴ 5 × 10⁴ to 1 × 10⁵, 1 × 10⁵ to 3 × 10⁵ 3 × 10⁵ to 5 × 10⁵, 5 × 10⁵ to 1 ×10⁶, 1 × 10⁶ to 3 × 10⁶, 3 × 10⁶ to 5 × 10⁶, 5 × 10⁶ to 1 × 10⁷, 1 × 10⁷ to 3 × 10⁷, 3 × 10⁷ to 5 × 10⁷, 5 × 10⁷, to 1 × 10⁸, 1 × 10⁸, to 3 × 10⁸, 3 × 10⁸, to 5 × 10⁸, 5 × 10⁸, to 1 × 10⁹, 1 × 10⁹ to 5 × 10⁹, or 5 × 10⁹ to 1 × 10¹⁰ placental cells. In certain embodiments, the pharmaceutical compositions provided herein comprises populations of placental derived adherent cells, that comprise 50% viable cells or more (that is, at least 50% of the cells in the population are functional or living). Preferably, at least 60% of the cells in the population are viable. More preferably, at least 70%, 80%, 90%, 95%, or 99% of the cells in the population in the pharmaceutical composition are viable.

### 4.3.2 Pharmaceutical Compositions

Populations of isolated placental cells, *e.g.,* PDACs, or populations of cells comprising the isolated placental cells, can be formulated into pharmaceutical compositions for use in vivo, e.g., in the methods of treatment provided herein. Such pharmaceutical compositions comprise a population of isolated placental cells, or a population of cells comprising isolated placental cells, in a pharmaceutically-acceptable carrier, e.g., a saline solution or other accepted physiologically-acceptable solution for in vivo administration. Pharmaceutical compositions comprising the isolated placental cells described herein can comprise any, or any combination, of the isolated placental cell populations, or isolated placental cells, described elsewhere herein. The pharmaceutical compositions can comprise fetal, maternal, or both fetal and maternal isolated placental cells. The pharmaceutical compositions provided herein can further comprise isolated placental cells obtained from a single individual or placenta, or from a plurality of individuals or placentae.

The pharmaceutical compositions provided herein can comprise any number of isolated placental cells. For example, a single unit dose of placental derived adherent cells can comprise about, at least, or no more than 1 × 10³, 3 × 10³, 5 × 10³, 1 × 10⁴, 3 × 10⁴, 5 × 10⁴, 1 × 10⁵, 3 × 10⁵, 5 × 10⁵, 1 × 10⁶, 3 × 10⁶, 5 × 10⁶, 1 × 10⁷, 3 × 10⁷, 5 × 10⁷, 1 × 10⁸, 3 × 10⁸, 5 × 10⁸, 1 × 10⁹, 5 × 10⁹, or 1 × 10¹⁰ placental cells or between 1 × 10³ to 3 × 10³, 3 × 10³ to 5 × 10³, 5 × 10³ to 1 × 10⁴, 1 × 10⁴ to 3 × 10⁴, 3 × 10⁴ to 5 × 10⁴, 5 × 10⁴ to 1 × 10⁵, 1 × 10⁵ to 3 × 10⁵, 3 × 10⁵ to 5 × 10⁵, 5 × 10⁵ to 1 × 10⁶, 1 × 10⁶ to 3 × 10⁶, 3 × 10⁶ to 5 × 10⁶, 5 × 10⁶ to 1 × 10⁷, 1 × 10⁷ to 3 × 10⁷, 3 × 10⁷ to 5 × 10⁷, 5 × 10⁷, to 1 × 10⁸, 1 × 10⁸, to 3 × 10⁸, 3 × 10⁸ to 5 × 10⁸, 5 × 10⁸, to 1 × 10⁹, 1 × 10⁹ to 5 × 10⁹, or 5 × 10⁹ to 1 × 10¹⁰ placental cells.

In certain embodiments, the pharmaceutical compositions provided herein are administered to a subject having diabetic peripheral neuropathy once. In certain embodiments, the pharmaceutical compositions provided herein are administered to a subject having diabetic peripheral neuropathy on multiple occasions, e.g., twice, three times, four times, five times, six times, seven times, eight times, nine times, ten times, or more than ten times. Intervals between dosages can be weekly, bi-weekly, monthly, bi-monthly or yearly. Intervals can also be irregular. Doses of placental stem cells administered according to such regimens include, but are not limited to, 1 × 10³, 3 × 10³, 5 × 10³, 1 × 10⁴, 3 × 10⁴, 5 × 10⁴ 1 × 10⁵, 3 × 10⁵, 5 × 10 ⁵ 1 × 10⁶, 3 × 10⁶, 5 × 10⁶, 1 × 10⁷, 3 × 10⁷, 5 × 10⁷, 1 × 10⁸, 3 × 10⁸, 5 × 10⁸, 1 × 10⁹, 5 × 10⁹ , or 1 × 10¹⁰ placental cells or between 1 × 10³ to 3 × 10³, 3 × 10³ to 5 × 10³, 5 × 10³ to 1 × 10⁴, 1 × 10⁴ to 3 × 10⁴, 3 × 10⁴ to 5 × 10⁴ 5 × 10⁴ to 1 × 10⁵, 1 × 10⁵ to 3 × 10⁵ 3 × 10 ⁵ to 5 × 10⁵, 5 × 10⁵ to 1 × 10⁶, 1 × 10⁶ to 3 × 10⁶, 3 × 10⁶ to 5 × 10⁶, 5 × 10⁶ to 1 × 10⁷, 1 × 10 ⁷ to 3 × 10⁷, 3 × 10⁷, to 5 × 10⁷, 5 × 10⁷ to 1 × 10⁸, 1 × 10⁸ to 3 × 10⁸, 3 × 10⁸ to 5 × 10⁸, 5 × 10⁸ to 1 × 10⁹, 1 × 10⁹ to 5 × 10⁹, or 5 × 10⁹ to 1 × 10¹⁰ placental stem cells. In a specific embodiment, the dose of placental stem cells in a pharmaceutical composition is 1 × 10³ placental stem cells. In another specific embodiment, the dose of placental stem cells in a pharmaceutical composition is 3 × 10³ placental stem cells. In another specific embodiment, the dose of placental stem cells in a pharmaceutical composition is 3 × 10⁴ placental stem cells. In another specific embodiment, the dose of placental stem cells in a pharmaceutical composition is 3 × 10⁵ placental stem cells. In another specific embodiment, the dose of placental stem cells in a pharmaceutical composition is 1 × 10⁶ placental stem cells. In another specific embodiment, the dose of placental stem cells in a pharmaceutical composition is 3 × 10⁶ placental stem cells. In another specific embodiment, the dose of placental stem cells in a pharmaceutical composition is 3 × 10⁷ placental stem cells.

In certain embodiments, a pharmaceutical composition comprising placental stem cells (e.g., CD10+, CD105+, CD200+, CD34- placental stem cells) is administered to a subject having diabetic peripheral neuropathy once as a single dose. In certain embodiments, a pharmaceutical composition comprising placental stem cells (e.g., CD10+, CD105+, CD200+, CD34- placental stem cells) is administered to a subject having diabetic peripheral neuropathy as a single dose followed by a second dose about 1 week later. In certain embodiments, a pharmaceutical composition comprising placental stem cells (e.g., CD10+, CD105+, CD200+, CD34- placental stem cells) is administered to a subject having diabetic peripheral neuropathy as a single dose followed by a second dose about 1 week later and a third dose about one week after that (i.e., about two weeks after the initial administration). Doses of placental stem cells administered according to such regimens include, but are not limited to, 1 × 10³, 3 × 10³, 5 × 10³, 1 × 10⁴, 3 × 10⁴, 5 × 10⁴, 1 × 10⁵, 3 × 10⁵, 5 × 10⁵, 1 × 10⁶, 3 × 10⁶, 5 ×10⁶, 1 × 10⁷, 3 × 10⁷, 5 × 10⁷, 1 × 10⁸, 3 × 10⁸, 5 × 10⁸, 1 × 10⁹, 5 × 10⁹, or 1 × 10¹⁰ placental cells or between 1 × 10³ to 3 × 10³, 3 × 10³ to 5 × 10³ 5 × 10³, to 1 × 10⁴, 1 × 10⁴ to 3 × 10⁴ 3 × 10⁴ to 5 × 10⁴ 5 × 10⁴ to 1 × 10⁵, 1 × 10⁵ to 3 × 10⁵, 3 × 10⁵ to 5 × 10⁵, 5 × 10⁵ to 1 × 10⁶, 1 × 10⁶ to 3 × 10⁶, 3 × 10⁶ to 5 × 10⁶, 5 × 10⁶ to 1 × 10⁷, 1 × 10⁷ to 3 × 10⁷, 3 × 10⁷ to 5 × 10⁷, 5 × 10⁷, to 1 × 10⁸, 1 × 10⁸, to 3 × 10⁸, 3 × 10⁸ to 5 × 10⁸, 5 × 10⁸, to 1 × 10⁹, 1 × 10⁹ to 5 × 10⁹, or 5 × 10⁹ to 1 × 10¹⁰ placental stem cells. In a specific embodiment, the dose of placental stem cells in a pharmaceutical composition is 1 × 10³ placental stem cells. In another specific embodiment, the dose of placental stem cells in a pharmaceutical composition is 3 × 10³ placental stem cells. In another specific embodiment, the dose of placental stem cells in a pharmaceutical composition is 3 × 10⁴ placental stem cells. In another specific embodiment, the dose of placental stem cells in a pharmaceutical composition is 3 × 10⁵ placental stem cells. In another specific embodiment, the dose of placental stem cells in a pharmaceutical composition is 1 × 10⁶ placental stem cells. In another specific embodiment, the dose of placental stem cells in a pharmaceutical composition is 3 × 10⁶ placental stem cells. In another specific embodiment, the dose of placental stem cells in a pharmaceutical composition is 3 × 10⁷ placental stem cells.

In certain embodiments, a pharmaceutical composition comprising placental stem cells (e.g., CD10+, CD105+, CD200+, CD34- placental stem cells) is administered to a subject having diabetic peripheral neuropathy as a single dose followed by a second dose about 1 month later (e.g., about 27, 28, 29, 30, 31, 32, or 33 days after the initial dose). In certain embodiments, a pharmaceutical composition comprising placental stem cells (e.g., CD10+, CD105+, CD200+, CD34- placental stem cells) is administered to a subject having diabetic peripheral neuropathy as a single dose followed by a second dose about 1 month later and a third dose about one month after that (i.e., about two months after the initial administration, e.g., on or about day 55, 56, 57, 58, 59, 60, 61, 62, 63, or 64 following the initial administration). Doses of placental stem cells administered according to such regimens include, but are not limited to, 1 × 10³, 3 × 10³, 5 × 10³, 1 × 10⁴, 3 × 10⁴, 5 × 10⁴, 1 × 10⁵, 3 × 10⁵, 5 × 10⁵, 1 × 10⁶, 3 × 10⁶, 5 × 10⁶, 1 × 10⁷, 3 × 10⁷, 5 × 10⁷, 1 × 10⁸, 3 × 10⁸, 5 × 10⁸, 1 × 10⁹, 5 × 10⁹, or 1 × 10¹⁰ placental cells or between 1 × 10³ to 3 × 10³, 3 × 10³ to 5 × 10³ 5 × 10³, to 1 × 10⁴, 1 × 10⁴ to 3 × 10⁴ 3 × 10⁴ to 5 × 10⁴, 5 × 10⁴ to 1 × 10⁵, 1 × 10⁵ to 3 × 10⁵, 3 × 10⁵ to 5 × 10⁵, 5 × 10⁵ to 1 × 10⁶, 1 × 10⁶ to 3 × 10⁶, 3 × 10⁶ to 5 × 10⁶, 5 × 10⁶ to 1 × 10⁷, 1 × 10⁷ to 3 × 10⁷, 3 × 10⁷ to 5 × 10⁷, 5 × 10⁷, to 1 × 10⁸, 1 × 10⁸, to 3 × 10⁸, 3 × 10⁸ to 5 × 10⁸, 5 × 10⁸, to 1 × 10⁹, 1 × 10⁹ to 5 × 10⁹, or 5 × 10⁹ to 1 × 10¹⁰ placental stem cells. In a specific embodiment, the dose of placental stem cells in a pharmaceutical composition is 1 × 10³ placental stem cells. In another specific embodiment, the dose of placental stem cells in a pharmaceutical composition is 3 × 10³ placental stem cells. In another specific embodiment, the dose of placental stem cells in a pharmaceutical composition is 3 × 10⁴ placental stem cells. In another specific embodiment, the dose of placental stem cells in a pharmaceutical composition is 3 × 10⁵ placental stem cells. In another specific embodiment, the dose of placental stem cells in a pharmaceutical composition is 1 × 10⁶ placental stem cells. In another specific embodiment, the dose of placental stem cells in a pharmaceutical composition is 3 × 10⁶ placental stem cells. In another specific embodiment, the dose of placental stem cells in a pharmaceutical composition is 3 × 10⁷ placental stem cells.

The pharmaceutical compositions provided herein comprise populations of cells that comprise 50% viable cells or more (that is, at least 50% of the cells in the population are functional or living). Preferably, at least 60% of the cells in the population are viable. More preferably, at least 70%, 80%, 90%, 95%, or 99% of the cells in the population in the pharmaceutical composition are viable.

The pharmaceutical compositions provided herein can comprise one or more compounds that, e.g., facilitate engraftment (e.g., anti-T-cell receptor antibodies, an immunosuppressant, or the like); stabilizers such as albumin, dextran 40, gelatin, hydroxyethyl starch, plasmalyte, and the like.

When formulated as an injectable solution, in one embodiment, the pharmaceutical composition comprises about 1% to 1.5% HSA and about 2.5% dextran. In a preferred embodiment, the pharmaceutical composition comprises from about 5 × 106 cells per milliliter to about 2 × 10⁷ cells per milliliter in a solution comprising 5% HSA and 10% dextran, optionally comprising an immunosuppressant, e.g., cyclosporine A at, e.g., 10 mg/kg.

In other embodiments, the pharmaceutical composition, e.g., a solution, comprises a plurality of cells, e.g., isolated placental cells, for example, placental stem cells or placental multipotent cells, wherein said pharmaceutical composition comprises between about 1.0 ±0.3 × 10⁶ cells per milliliter to about 5.0 ± 1.5 × 10⁶ cells per milliliter. In other embodiments, the pharmaceutical composition comprises between about 1.5 × 10⁶ cells per milliliter to about 3.75 × 10⁶ cells per milliliter. In other embodiments, the pharmaceutical composition comprises between about 1 × 10⁶ cells/mL to about 50 × 10⁶ cells/mL, about 1 × 10⁶ cells/mL to about 40 × 10⁶ cells/mL, about 1 × 10⁶ cells/mL to about 30 × 10⁶ cells/mL, about 1 × 10⁶ cells/mL to about 20 × 10⁶ cells/mL, about 1 × 10⁶ cells/mL to about 15 × 10⁶ cells/mL, or about 1 × 10⁶ cells/mL to about 10 × 10⁶ cells/mL. In certain embodiments, the pharmaceutical composition comprises no visible cell clumps (i.e., no macro cell clumps), or substantially no such visible clumps. As used herein, "macro cell clumps" means an aggregation of cells visible without magnification, e.g., visible to the naked eye, and generally refers to a cell aggregation larger than about 150 microns In some embodiments, the pharmaceutical composition comprises about 2.5%, 3.0%, 3.5%, 4.0%, 4.5%, 5.0%, 5.5%, 6.0%, 6.5%, 7.0%, 7.5% 8.0%, 8.5%, 9.0%, 9.5% or 10% dextran, e.g., dextran-40. In a specific embodiment, said composition comprises about 7.5% to about 9% dextran-40. In a specific embodiment, said composition comprises about 5.5 % dextran-40. In certain embodiments, the pharmaceutical composition comprises from about 1% to about 15% human serum albumin (HSA). In specific embodiments, the pharmaceutical composition comprises about 1%, 2%, 3%, 4%, 5%, 65, 75, 8%, 9%, 10%, 11%, 12%, 13%, 14% or 15% HSA. In a specific embodiment, said cells have been cryopreserved and thawed. In another specific embodiment, said cells have been filtered through a 70 µM to 100 µM filter. In another specific embodiment, said composition comprises no visible cell clumps. In another specific embodiment, said composition comprises fewer than about 200 cell clumps per 106 cells, wherein said cell clumps are visible only under a microscope, e.g., a light microscope. In another specific embodiment, said composition comprises fewer than about 150 cell clumps per 10⁶ cells, wherein said cell clumps are visible only under a microscope, e.g., a light microscope. In another specific embodiment, said composition comprises fewer than about 100 cell clumps per 10⁶ cells, wherein said cell clumps are visible only under a microscope, e.g., a light microscope.

In a specific embodiment, the pharmaceutical composition comprises about 1.0 ±0.3 × 10⁶ cells per milliliter, about 5.5% dextran-40 (w/v) , about 10% HSA (w/v), and about 5% DMSO (v/v). In another specific embodiment, a pharmaceutical composition comprising placental stem cells provided herein comprises about 5.75% dextran 40, about 10% human serum albumin, and about 2.5% DMSO.

In other embodiments, the pharmaceutical composition comprises a plurality of cells, e.g., a plurality of isolated placental cells in a solution comprising 10% dextran-40, wherein the pharmaceutical composition comprises between about 1.0 ± 0.3 × 10⁶ cells per milliliter to about 5.0 ±1.5 × 10⁶ cells per milliliter, and wherein said composition comprises no cell clumps visible with the unaided eye (i.e., comprises no macro cell clumps). In some embodiments, the pharmaceutical composition comprises between about 1.5 × 10⁶ cells per milliliter to about 3.75 × 10⁶ cells per milliliter. In a specific embodiment, said cells have been cryopreserved and thawed. In another specific embodiment, said cells have been filtered through a 70 µM to 100 µM filter. In another specific embodiment, said composition comprises fewer than about 200 micro cell clumps (that is, cell clumps visible only with magnification) per 10⁶ cells. In another specific embodiment, the pharmaceutical composition comprises fewer than about 150 micro cell clumps per 10⁶ cells. In another specific embodiment, the pharmaceutical composition comprises fewer than about 100 micro cell clumps per 10⁶ cells. In another specific embodiment, the pharmaceutical composition comprises less than 15%, 14%, 13%, 12%, 11%, 10%, 9%, 8%, 7%, 6%, 5%, 4%, 3%, or 2% DMSO, or less than 1%, 0.9%, 0.8%, 0.7%, 0.6%, 0.5%, 0.4%, 0.3%, 0.2%, or 0.1% DMSO.

Further provided herein are compositions comprising cells, wherein said compositions are produced by one of the methods disclosed herein. For example, in one embodiment, the pharmaceutical composition comprises cells, wherein the pharmaceutical composition is produced by a method comprising filtering a solution comprising placental cells, e.g., placental stem cells or placental multipotent cells, to form a filtered cell-containing solution; diluting the filtered cell-containing solution with a first solution to about 1 to 50 × 10⁶, 1 to 40 × 10⁶, 1 to 30 × 10⁶, 1 to 20 × 10⁶, 1 to 15 × 10⁶, or 1 to 10 × 10⁶ cells per milliliter, e.g., prior to cryopreservation; and diluting the resulting filtered cell-containing solution with a second solution comprising dextran, but not comprising human serum albumin (HSA) to produce said composition. In certain embodiments, said diluting is to no more than about 15 × 10⁶ cells per milliliter. In certain embodiments, said diluting is to no more than about 10 ± 3 × 10⁶ cells per milliliter. In certain embodiments, said diluting is to no more than about 7.5 × 10⁶ cells per milliliter. In other certain embodiments, if the filtered cell-containing solution, prior to the dilution, comprises less than about 15 × 10⁶ cells per milliliter, filtration is optional. In other certain embodiments, if the filtered cell-containing solution, prior to the dilution, comprises less than about 10 ± 3 × 10⁶ cells per milliliter, filtration is optional. In other certain embodiments, if the filtered cell-containing solution, prior to the dilution, comprises less than about 7.5 × 10⁶ cells per milliliter, filtration is optional.

In a specific embodiment, the cells are cryopreserved between said diluting with a first dilution solution and said diluting with said second dilution solution. In another specific embodiment, the first dilution solution comprises dextran and HSA. The dextran in the first dilution solution or second dilution solution can be dextran of any molecular weight, e.g., dextran having a molecular weight of from about 10 kDa to about 150 kDa. In some embodiments, said dextran in said first dilution solution or said second solution is about 2.5%, 3.0%, 3.5%, 4.0%, 4.5%, 5.0%, 5.5%, 6.0%, 6.5%, 7.0%, 7.5% 8.0%, 8.5%, 9.0%, 9.5% or 10% dextran. In another specific embodiment, the dextran in said first dilution solution or said second dilution solution is dextran-40. In another specific embodiment, the dextran in said first dilution solution and said second dilution solution is dextran-40. In another specific embodiment, said dextran-40 in said first dilution solution is 5.0% dextran-40. In another specific embodiment, said dextran-40 in said first dilution solution is 5.5% dextran-40. In another specific embodiment, said dextran-40 in said second dilution solution is 10% dextran-40. In another specific embodiment, said HSA in said solution comprising HSA is 1 to 15 % HSA. In another specific embodiment, said HSA in said solution comprising HSA is about 1%, 2%, 3%, 4%, 5%, 6%, 7%, 8%, 9%, 10%, 11%, 12%, 13%, 14% or 15 % HSA. In another specific embodiment, said HSA in said solution comprising HSA is 10% HSA. In another specific embodiment, said first dilution solution comprises HSA. In another specific embodiment, said HSA in said first dilution solution is 10% HSA. In another specific embodiment, said first dilution solution comprises a cryoprotectant. In another specific embodiment, said cryoprotectant is DMSO. In another specific embodiment, said dextran-40 in said second dilution solution is about 10% dextran-40. In another specific embodiment, said composition comprising cells comprises about 7.5% to about 9% dextran. In another specific embodiment, the pharmaceutical composition comprises from about 1.0 ±0.3 × 10⁶ cells per milliliter to about 5.0 ±1.5 × 10⁶ cells per milliliter. In another specific embodiment, the pharmaceutical composition comprises from about 1.5 × 10⁶ cells per milliliter to about 3.75 × 10⁶ cells per milliliter.

In another embodiment, the pharmaceutical composition is made by a method comprising (a) filtering a cell-containing solution comprising placental cells, e.g., placental stem cells or placental multipotent cells, prior to cryopreservation to produce a filtered cell-containing solution; (b) cryopreserving the cells in the filtered cell-containing solution at about 1 to 50 × 10⁶, 1 to 40 × 10⁶, 1 to 30 × 10⁶, 1 to 20 × 10⁶, 1 to 15 × 10⁶, or 1 to 10 × 10⁶ cells per milliliter; (c) thawing the cells; and (d) diluting the filtered cell-containing solution about 1:1 to about 1:11 (v/v) with a dextran-40 solution. In certain embodiments, if the number of cells is less than about 10 ± 3 × 10⁶ cells per milliliter prior to step (a), filtration is optional. In another specific embodiment, the cells in step (b) are cryopreserved at about 10 ± 3 × 10⁶ cells per milliliter. In another specific embodiment, the cells in step (b) are cryopreserved in a solution comprising about 5% to about 10% dextran-40 and HSA. In certain embodiments, said diluting in step (b) is to no more than about 15 × 10⁶ cells per milliliter.

In another embodiment, the pharmaceutical composition is made by a method comprising: (a) suspending placental cells, e.g., placental stem cells or placental multipotent cells, in a 5.5% dextran-40 solution that comprises 10% HSA to form a cell-containing solution; (b) filtering the cell-containing solution through a 70 µM filter; (c) diluting the cell-containing solution with a solution comprising 5.5% dextran-40, 10% HSA, and 5% DMSO to about 1 to 50 × 10⁶, 1 to 40 × 10⁶, 1 to 30 × 10⁶, 1 to 20 × 10⁶, 1 to 15 × 10⁶, or 1 to 10 × 10⁶ cells per milliliter; (d) cryopreserving the cells; (e) thawing the cells; and (f) diluting the cell-containing solution 1:1 to 1:11 (v/v) with 10% dextran-40. In certain embodiments, said diluting in step (c) is to no more than about 15 × 10⁶ cells per milliliter. In certain embodiments, said diluting in step (c) is to no more than about 10 ± 3 × 10⁶ cells/mL. In certain embodiments, said diluting in step (c) is to no more than about 7.5 × 10⁶ cells/mL.

In another embodiment, the composition comprising cells is made by a method comprising: (a) centrifuging a plurality of cells to collect the cells; (b) resuspending the cells in 5.5% dextran-40; (c) centrifuging the cells to collect the cells; (d) resuspending the cells in a 5.5% dextran-40 solution that comprises 10% HSA; (e) filtering the cells through a 70 µM filter; (f) diluting the cells in 5.5% dextran-40, 10% HSA, and 5% DMSO to about 1 to 50 × 10⁶, 1 to 40 × 10⁶, 1 to 30 × 10⁶, 1 to 20 × 10⁶, 1 to 15 × 10⁶, or 1 to 10 × 10⁶ cells per milliliter; (g) cryopreserving the cells; (h) thawing the cells; and (i) diluting the cells 1:1 to 1:11 (v/v) with 10% dextran-40. In certain embodiments, said diluting in step (f) is to no more than about 15 × 10⁶ cells per milliliter. In certain embodiments, said diluting in step (f) is to no more than about 10 ± 3 × 10⁶ cells/mL. In certain embodiments, said diluting in step (f) is to no more than about 7.5 × 10⁶ cells/mL. In other certain embodiments, if the number of cells is less than about 10 ± 3 × 10⁶ cells per milliliter, filtration is optional.

The compositions, e.g., pharmaceutical compositions comprising the isolated placental cells, described herein can comprise any of the isolated placental cells described herein.

Other injectable formulations, suitable for the administration of cellular products, may be used.

In one embodiment, the pharmaceutical composition comprises isolated placental cells that are substantially, or completely, non-maternal in origin, that is, have the fetal genotype; e.g., at least about 90%, 95%, 98%, 99% or about 100% are non-maternal in origin. For example, in one embodiment a pharmaceutical composition comprises a population of isolated placental cells that are CD200+ and HLA-G-; CD73+, CD105+, and CD200+; CD200+ and OCT-4+; CD73+, CD105+ and HLA-G-; CD73+ and CD105+ and facilitate the formation of one or more embryoid-like bodies in a population of placental cells comprising said population of isolated placental cell when said population of placental cells is cultured under conditions that allow the formation of an embryoid-like body; or OCT-4+ and facilitate the formation of one or more embryoid-like bodies in a population of placental cells comprising said population of isolated placental cell when said population of placental cells is cultured under conditions that allow the formation of an embryoid-like body; or a combination of the foregoing, wherein at least 70%, 80%, 90%, 95% or 99% of said isolated placental cells are non-maternal in origin. In another embodiment, a pharmaceutical composition comprises a population of isolated placental cells that are CD10+, CD105+ and CD34-; CD10+, CD105+, CD200+ and CD34-; CD10+, CD105+, CD200+, CD34- and at least one of CD90+ or CD45-; CD10+, CD90+, CD105+, CD200+, CD34- and CD45-; CD10+, CD90+, CD105+, CD200+, CD34- and CD45-; CD200+ and HLA-G-; CD73+, CD105+, and CD200+; CD200+ and OCT-4+; CD73+, CD105+ and HLA-G-; CD73+ and CD105+ and facilitate the formation of one or more embryoid-like bodies in a population of placental cells comprising said isolated placental cells when said population of placental cells is cultured under conditions that allow the formation of an embryoid-like body; OCT-4+ and facilitate the formation of one or more embryoid-like bodies in a population of placental cells comprising said isolated placental cells when said population of placental cells is cultured under conditions that allow the formation of an embryoid-like body; or one or more of CD117―, CD133-, KDR-, CD80-, CD86-, HLA-A,B,C+, HLA-DP,DQ,DR- and/or PDL1+; or a combination of the foregoing, wherein at least 70%, 80%, 90%, 95% or 99% of said isolated placental cells are non-maternal in origin. In a specific embodiment, the pharmaceutical composition additionally comprises a stem cell that is not obtained from a placenta.

Isolated placental cells in the compositions, e.g., pharmaceutical compositions, provided herein, can comprise placental cells derived from a single donor, or from multiple donors. The isolated placental cells can be completely HLA-matched to an intended recipient, or partially or completely HLA-mismatched.

### 4.3.3 Matrices Comprising Isolated Placental Cells

Further provided herein are compositions comprising matrices, hydrogels, scaffolds, and the like that comprise a placental cell, or a population of isolated placental cells. Such compositions can be used in the place of, or in addition to, cells in liquid suspension.

The isolated placental cells described herein can be seeded onto a natural matrix, e.g., a placental biomaterial such as an amniotic membrane material. Such an amniotic membrane material can be, e.g., amniotic membrane dissected directly from a mammalian placenta; fixed or heat-treated amniotic membrane, substantially dry (i.e., <20% H2O) amniotic membrane, chorionic membrane, substantially dry chorionic membrane, substantially dry amniotic and chorionic membrane, and the like. Preferred placental biomaterials on which isolated placental cells can be seeded are described in Hariri, U.S. Application Publication No. 2004/0048796, the disclosure of which is incorporated herein by reference in its entirety.

The isolated placental cells described herein can be suspended in a hydrogel solution suitable for, e.g., injection. Suitable hydrogels for such compositions include self-assembling peptides, such as RAD16. In one embodiment, a hydrogel solution comprising the cells can be allowed to harden, for instance in a mold, to form a matrix having cells dispersed therein for implantation. Isolated placental cells in such a matrix can also be cultured so that the cells are mitotically expanded prior to implantation. The hydrogel is, e.g., an organic polymer (natural or synthetic) that is cross-linked via covalent, ionic, or hydrogen bonds to create a three-dimensional open-lattice structure that entraps water molecules to form a gel. Hydrogel-forming materials include polysaccharides such as alginate and salts thereof, peptides, polyphosphazines, and polyacrylates, which are crosslinked ionically, or block polymers such as polyethylene oxide-polypropylene glycol block copolymers which are crosslinked by temperature or pH, respectively. In some embodiments, the hydrogel or matrix is biodegradable.

In some embodiments, the formulation comprises an in situ polymerizable gel (see., e.g., U.S. Patent Application Publication 2002/0022676, the disclosure of which is incorporated herein by reference in its entirety; Anseth et al., J. Control Release, 78(1-3):199-209 (2002); Wang et al., Biomaterials, 24(22):3969-80 (2003).

In some embodiments, the polymers are at least partially soluble in aqueous solutions, such as water, buffered salt solutions, or aqueous alcohol solutions, that have charged side groups, or a monovalent ionic salt thereof. Examples of polymers having acidic side groups that can be reacted with cations are poly(phosphazenes), poly(acrylic acids), poly(methacrylic acids), copolymers of acrylic acid and methacrylic acid, poly(vinyl acetate), and sulfonated polymers, such as sulfonated polystyrene. Copolymers having acidic side groups formed by reaction of acrylic or methacrylic acid and vinyl ether monomers or polymers can also be used. Examples of acidic groups are carboxylic acid groups, sulfonic acid groups, halogenated (preferably fluorinated) alcohol groups, phenolic OH groups, and acidic OH groups.

In a specific embodiment, the matrix is a felt, which can be composed of a multifilament yarn made from a bioabsorbable material, e.g., PGA, PLA, PCL copolymers or blends, or hyaluronic acid. The yarn is made into a felt using standard textile processing techniques consisting of crimping, cutting, carding and needling. In another preferred embodiment the cells of the invention are seeded onto foam scaffolds that may be composite structures. In addition, the three-dimensional framework may be molded into a useful shape, such as a specific structure in the body to be repaired, replaced, or augmented. Other examples of scaffolds that can be used include nonwoven mats, porous foams, or self assembling peptides. Nonwoven mats can be formed using fibers comprised of a synthetic absorbable copolymer of glycolic and lactic acids (e.g., PGA/PLA) (VICRYL, Ethicon, Inc., Somerville, N.J.). Foams, composed of, e.g., poly(ε-caprolactone)/poly(glycolic acid) (PCL/PGA) copolymer, formed by processes such as freeze-drying, or lyophilization (see, e.g., U.S. Pat. No. 6,355,699), can also be used as scaffolds.

The isolated placental cells described herein or co-cultures thereof can be seeded onto a three-dimensional framework or scaffold and implanted in vivo. Such a framework can be implanted in combination with any one or more growth factors, cells, drugs or other components that, e.g., stimulate tissue formation.

Examples of scaffolds that can be used include nonwoven mats, porous foams, or self assembling peptides. Nonwoven mats can be formed using fibers comprised of a synthetic absorbable copolymer of glycolic and lactic acids (e.g., PGA/PLA) (VICRYL, Ethicon, Inc., Somerville, N.J.). Foams, composed of, e.g., poly(ε-caprolactone)/poly(glycolic acid) (PCL/PGA) copolymer, formed by processes such as freeze-drying, or lyophilization (see, e.g., U.S. Pat. No. 6,355,699), can also be used as scaffolds.

In another embodiment, isolated placental cells can be seeded onto, or contacted with, a felt, which can be, e.g., composed of a multifilament yarn made from a bioabsorbable material such as PGA, PLA, PCL copolymers or blends, or hyaluronic acid.

The isolated placental cells provided herein can, in another embodiment, be seeded onto foam scaffolds that may be composite structures. Such foam scaffolds can be molded into a useful shape, such as that of a portion of a specific structure in the body to be repaired, replaced or augmented. In some embodiments, the framework is treated, e.g., with 0.1M acetic acid followed by incubation in polylysine, PBS, and/or collagen, prior to inoculation of the cells in order to enhance cell attachment. External surfaces of a matrix may be modified to improve the attachment or growth of cells and differentiation of tissue, such as by plasma-coating the matrix, or addition of one or more proteins (e.g., collagens, elastic fibers, reticular fibers), glycoproteins, glycosaminoglycans (e.g., heparin sulfate, chondroitin-4-sulfate, chondroitin-6-sulfate, dermatan sulfate, keratin sulfate, etc.), a cellular matrix, and/or other materials such as, but not limited to, gelatin, alginates, agar, agarose, and plant gums, and the like.

In some embodiments, the scaffold comprises, or is treated with, materials that render it non-thrombogenic. These treatments and materials may also promote and sustain endothelial growth, migration, and extracellular matrix deposition. Examples of these materials and treatments include but are not limited to natural materials such as basement membrane proteins such as laminin and Type IV collagen, synthetic materials such as EPTFE, and segmented polyurethaneurea silicones, such as PURSPAN^{™} (The Polymer Technology Group, Inc., Berkeley, Calif.). The scaffold can also comprise anti-thrombotic agents such as heparin; the scaffolds can also be treated to alter the surface charge (e.g., coating with plasma) prior to seeding with isolated placental cells.

The placental cells (e.g., PDACs) provided herein can also be seeded onto, or contacted with, a physiologically-acceptable ceramic material including, but not limited to, mono-, di-, tri-, alpha-tri-, beta-tri-, and tetra-calcium phosphate, hydroxyapatite, fluoroapatites, calcium sulfates, calcium fluorides, calcium oxides, calcium carbonates, magnesium calcium phosphates, biologically active glasses such as BIOGLASS^{®}, and mixtures thereof. Porous biocompatible ceramic materials currently commercially available include SURGIBONE^{®} (CanMedica Corp., Canada), ENDOBON^{®} (Merck Biomaterial France, France), CEROS^{®} (Mathys, AG, Bettlach, Switzerland), and mineralized collagen bone grafting products such as HEALOS^{™} (DePuy, Inc., Raynham, MA) and VITOSS^{®}, RHAKOSS^{™}, and CORTOSS^{®} (Orthovita, Malvern, Pa.). The framework can be a mixture, blend or composite of natural and/or synthetic materials.

In one embodiment, the isolated placental cells are seeded onto, or contacted with, a suitable scaffold at about 0.5 × 10⁶ to about 8 × 10⁶ cells/mL.

### 5. EXAMPLES

### 5.1 EXAMPLE 1: METHOD OF TREATMENT

### 5.1.1 Treatment of DPN Using Placental Stem Cells

A 52 year old male with type I diabetes presents with numbness and pain in his left leg. A diagnosis of diabetic peripheral neuropathy is made. After diagnosis, the subject is treated with CD10+, CD34-, CD105+, CD200+ placental stem cells according to the following regimen: 3 × 10⁷ CD10+, CD34-, CD105+, CD200+ placental stem cells are administered intramuscularly on a monthly basis for three consecutive months. The individual is monitored over the next 24 months for signs of improvement in any symptom of the DPN. Therapeutic effectiveness is established if any of the symptoms of the DPN improve during the monitoring period, including improvement in epidermal nerve fiber density as measured by qusntification and qualification of epidermal nerve fibers in a skin biopsy.

### 5.2 EXAMPLE 2: DPN TREATMENT PROTOCOL

Subjects having diabetic peripheral neuropathy (DPN), at least 18 years of age, are treated with CD10⁺, CD34⁻, CD105+, CD200⁺ placental stem cells. Subject Group I: 3 × 10⁶ CD10+, CD34⁻, CD105+, CD200⁺ placental stem cells are administered intramuscularly on days 1 (the first day of treatment), 29, and 57. Subject Group II: 3 × 10⁷ CD10⁺, CD34⁻, CD105+, CD200⁺ placental stem cells are administered intramuscularly on days 1 (the first day of treatment), 29, and 57. Subject Group III: placebo is administered intramuscularly on days 1 (the first day of treatment), 29, and 57. Each dose of placental stem cells is administered as fifteen 0.30 ml injections, and administration is below the knee and above the ankle of the subject.

### Clinical Endpoints

A primary clinical endpoint for efficacy of CD10+, CD34⁻, CD105+, CD200⁺ placental stem cells for treating DPN can be improvement in epidermal nerve fiber density as measured by quantification and qualification of epidermal nerve fibers in a skin biopsy. An increase in the number/density of nerve fibers is indicative of improving neuropathy.

### Subject Selection

The following eligibility criteria may be used to select subjects for whom treatment with CD10+, CD34⁻, CD105+, CD200⁺ placental stem cells is considered appropriate. All relevant medical and non-medical conditions are taken into consideration when deciding whether this treatment protocol is suitable for a particular subject.

Subjects should meet the following conditions to be eligible for the treatment protocol:
- Males and females, at least 18 years of age or older.
- Diabetes mellitus (DM) Type 2 as defined by the American Diabetes Association (ADA) or World Health Organization (WHO) criteria.
- Meet established criteria for diabetic peripheral neuropathy (DPN) due to Type 2 diabetes with the following: (a) Abnormal symptoms (NTSS-6 ≥ 6 points (total score) or ≥ 2.0 points for one or more symptoms), and (b) Abnormal signs (UENS score of 2-24 and/or NIS-LL score of 2-10)..
- A female of childbearing potential must have a negative serum pregnancy test at screening and a negative urine pregnancy test prior to treatment with study therapy. In addition, sexually active Females of Child Bearing Potential (FCBP) must agree to use 2 of the following adequate forms of contraception methods simultaneously such as: oral, injectable, or implantable hormonal contraception, tubal ligation, intrauterine device (IUD), barrier contraceptive with spermicide or vasectomized partner for the duration of the study.
- Males (including those who have had a vasectomy) must agree to use barrier contraception (latex condoms) when engaging in sexual activity with FCBP for the duration of the study.

Subjects having one or more of the following conditions can be excluded from the treatment protocol:
- Any significant medical condition, laboratory abnormality, or psychiatric illness that would prevent the subject from participating in the study.
- Other causes of neuropathy in diabetic subjects: chronic inflammatory demyelinating polyneuropathy; neuropathy due to vitamin B12 deficiency, hypothyroidism, and uremia syndrome; or neuropathy due to entrapment or trauma.
- A reversible course of acute painful diabetic neuropathy syndrome: treatment-induced diabetic neuropathy that presents in the setting of rapid glycemic control; diabetic neuropathic cachexia; and diabetic anorexia, a diabetic neuropathy that is seen with intentional weight loss.
- History of a prior diagnosis of severe peripheral arterial disease (PAD).
- Thrombocytopenia and coagulopathy, to avoid severe bruising or bleeding due to multiple intramuscular (IM) injections.
- Any condition including the presence of laboratory abnormalities that places the subject at unacceptable risk if he or she were to participate in the study.
- Any condition that confounds the ability to interpret data from the study.
- Subjects who are taking opioids for the treatment of DPN.
- Pregnant or lactating females.
- Subjects with a body mass index > 40 kg/m2 at screening.
- Neuropathy resulting from a condition other than DM and/or significant co-morbid neurological diseases (eg, Parkinson's disease, epilepsy, multiple sclerosis, alcoholic peripheral neuropathy), or exposure to agents suspected to cause symptoms of neuropathy (such as but not limited to metronidazole, antituberculosis medications, and heavy metals.
- Advanced neuropathy as measured by the absence of sural sensory nerve action potential, or a UENS>24 and or a NIS-LL>10.
- History of a prior diagnosis of Critical Limb Ischemia.
- History of diabetic foot ulceration (at any time) and/or or undergoing a limb revascularization procedure(s) and/or amputation(s) due to diabetes mellitus (DM).
- Diagnosis of Type 1 DM and/or any of the following: diagnosis of DM prior to age 35 years; insulin required to treat DM within 1 year after DM diagnosis; history of diabetic Ketoacidosis.
- Aspartate Aminotransferase (AST), Alanine Aminotransferase (ALT), or alkaline phosphatase ≥ 2.5 × the upper limit of normal (ULN) at screening.
- Estimated glomerular filtration rate (eGFR) < 30 mL/min/1.73 m2 at Screening calculated using the Modification of Diet in Renal Disease Study equation or history of an abnormal eGFR < 60 and decline > 15 mL/min/1.73 m2 below normal in the past year.
- Bilirubin level > 2 mg/dL (unless subject has known Gilbert's disease) at screening.
- Untreated chronic infection or treatment of any infection with systemic antibiotics within 4 weeks prior to dosing with IP.
- Uncontrolled hypertension (defined as diastolic blood pressure > 100 mmHg or systolic blood pressure > 180 mmHg during screening at 2 independent measurements taken while subject is sitting and resting for at least 5 minutes).
- History of significant cardiac disorders including but not limited to malignant ventricular arrhythmia, CCS Class III-IV angina pectoris, myocardial infarction/ percutaneous coronary intervention (PCI) / coronary artery bypass graft (CABG) in the 6 months prior to signing the informed consent form (ICF), pending coronary revascularization in the following 3 months, transient ischemic attack/cerebrovascular accident in the 6 months prior to signing the informed consent form, and/or New York Heart Association [NYHA] Stage III or IV congestive heart failure. Note: Stable Canadian Cardiovascular Society (CCS) Class I-II angina is allowed.
- Poorly controlled DM (hemoglobin A1c > 10%) at screening.
- Untreated proliferative retinopathy at screening.
- Life expectancy less than 2 years due to concomitant illnesses.
- History of malignancy within 5 years except for the following circumstances: basal cell or squamous cell carcinoma of the skin, remote history of cancer now considered cured or positive Pap smear with subsequent negative follow-up.
- History of hypersensitivity to any of the components of bovine or porcine products, dextran 40, and dimethyl sulfoxide [DMSO]).
- Subject has received an investigational agent —an agent or device not approved by the US Food and Drug Administration (FDA) for marketed use in any indication— within 90 days (or 5 half-lives, whichever is longer) prior to treatment with study therapy or planned participation in another therapeutic study prior to the completion of this study or has received previous gene or cell therapy at any time.

### Clinical Outcome

Efficacy of the CD10⁺, CD34⁻, CD105⁺, CD200⁺ placental stem cells in treatment of DPN is confirmed if improvement in one or more clinical endpoints is demonstrated.

### Equivalents:

The present disclosure is not to be limited in scope by the specific embodiments described herein. Indeed, various modifications of the subject matter provided herein, in addition to those described, will become apparent to those skilled in the art from the foregoing description. Such modifications are intended to fall within the scope of the appended claims.

Various publications, patents and patent applications are cited herein, the disclosures of which are incorporated by reference in their entireties.

The following paragraphs describe embodiments of the invention.
1. A method of treating a subject having a diabetic peripheral neuropathy, comprising administering to the subject a composition comprising CD10⁺, CD34", CD105+, CD200+ placental stem cells.
2. The method of paragraph 1, wherein said diabetic peripheral neuropathy affects one or more of the hands, feet, arms, or legs of said subject.
3. The method of paragraph 1, wherein said diabetic peripheral neuropathy affects each of the hands, feet, arms, or legs of said subject.
4. The method of any one of paragraphs 1-3, wherein said composition comprising placental stem cells is administered intramuscularly.
5. The method of any one of paragraphs 1-4, wherein said composition comprises between 1 × 10⁵ to 1 × 10⁶, 1 × 10⁶ to 3 × 10⁶, 3 × 10⁶ to 5 × 10⁶, 5 × 10⁶ to 1 × 10⁷, 1 × 10⁷ to 3 × 10⁷, 3 × 10⁷ to 5 × 10⁷, 5 × 10⁷ to 1 × 10⁸, 1 × 10⁸ to 3 × 10⁸, 3 × 10⁸ to 5 × 10⁸, 5 × 10⁸ to 1 × 10⁹, 1 × 10⁹ to 5 × 10⁹, or 5 × 10⁹ to 1 × 10¹⁰ placental stem cells.
6. The method of any one of paragraphs 1-4, wherein said composition comprises about 1 × 10⁵, 3 × 10⁵, 5 × 10⁵, 1 × 10⁶, 3 × 10⁶, 5 × 10⁶, 1 × 10⁷, 3 × 10⁷, 5 × 10⁷, 1 × 10⁸, 3 × 10⁸, 5 × 10⁸, 1 × 10⁹, 5 × 10⁹, or 1 × 10¹⁰ placental stem cells.
7. The method of paragraph 6, wherein said composition comprises about 3 × 10⁶ placental stem cells.
8. The method of paragraph 6, wherein said composition comprises about 3 × 10⁷ placental stem cells.
9. The method of any one of paragraphs 1-8, wherein said treatment results in an increase in epidermal nerve fiber density in said subject.
10. The method of paragraph 9, wherein said increase in epidermal nerve fiber density in said subject is measured by skin biopsy.
11. The method of any one of paragraphs 1-10, wherein said treatment results in improvement in one or more symptoms of said diabetic peripheral neuropathy.
12. The method of paragraph 11, wherein said one or more symptoms is numbness or reduced ability to feel pain or temperature changes, a tingling or burning sensation in the limbs, sharp pains or cramps, increased sensitivity to touch, muscle weakness, loss of reflexes (e.g., in the ankle), loss of balance and/or coordination, and/or foot problems (such as ulcers, infections, deformities, and bone and joint pain).

## Claims

1. A composition comprising CD10⁺, CD34⁻, CD105⁺, CD200⁺ placental stem cells for use in method of treating a diabetic peripheral neuropathy in a subject, the method comprising administering the composition to the subject.

2. The composition for use according to claim 1, wherein said diabetic peripheral neuropathy affects one or more of the hands, feet, arms, or legs of said subject.

3. The composition for use according to claim 1, wherein said diabetic peripheral neuropathy affects each of the hands, feet, arms, or legs of said subject.

4. The composition for use according to any one of claims 1-3, wherein said composition comprising placental stem cells is administered intramuscularly.

5. The composition for use according to method of any one of claims 1-4, wherein said composition comprises between 1 × 10⁵ to 1 × 10⁶, 1 × 10⁶ to 3 × 10⁶, 3 × 10⁶ to 5 × 10⁶, 5 × 10⁶ to 1 × 10⁷, 1 × 10⁷ to 3 × 10⁷, 3 × 10⁷ to 5 × 10⁷, 5 × 10⁷ to 1 × 10⁸, 1 × 10⁸ to 3 × 10⁸, 3 × 10⁸ to 5 × 10⁸, 5 × 10⁸ to 1 × 10⁹, 1 × 10⁹ to 5 × 10⁹, or 5 × 10⁹ to 1 × 10¹⁰ placental stem cells.

6. The composition for use according to any one of claims 1-4, wherein said composition comprises about 1 × 10⁵, 3 × 10⁵, 5 × 10⁵, 1 × 10⁶, 3 × 10⁶, 5 × 10⁶, 1 × 10⁷, 3 × 10⁷, 5 × 10⁷, 1 × 10⁸, 3 × 10⁸, 5 × 10⁸, 1 × 10⁹, 5 × 10⁹, or 1 × 10¹⁰ placental stem cells.

7. The composition for use according to claim 6, wherein said composition comprises about 3 × 10⁶ placental stem cells.

8. The composition for use according to claim 6, wherein said composition comprises about 3 × 10⁷ placental stem cells.

9. The composition for use according to any one of claims 1-8, wherein said treatment results in an increase in epidermal nerve fiber density in said subject.

10. The composition for use according to claim 9, wherein said increase in epidermal nerve fiber density in said subject is measured by skin biopsy.

11. The composition for use according to any one of claims 1-10, wherein said treatment results in improvement in one or more symptoms of said diabetic peripheral neuropathy.

12. The composition for use according to claim 11, wherein said one or more symptoms is numbness or reduced ability to feel pain or temperature changes, a tingling or burning sensation in the limbs, sharp pains or cramps, increased sensitivity to touch, muscle weakness, loss of reflexes (e.g., in the ankle), loss of balance and/or coordination, and/or foot problems (such as ulcers, infections, deformities, and bone and joint pain).
